# EUROPEAN PATENT APPLICATION

(11) **EP 2 789 688 A1**
(43) Date of publication of application: **15.10.2014**
(21) Application number: 12856362.4
(22) Date of filing: 07.12.2012
(51) Int. Cl.: C12N 15/09, C12Q 1/68

(54) **METHOD FOR DETECTING NUCLEOTIDE MUTATION, AND DETECTION KIT**

(30) Priority: 09.12.2011 JP 2011269967
(71) Applicant: Eisai R&D Management Co., Ltd., Tokyo 112-8088 (JP)
(72) Inventor: FUJIKAWA, Toshihiko, Inashiki-gun Ibaraki 3001155 (JP); OSAWA, Masako, Inashiki-gun Ibaraki 3001155 (JP)
(74) Representative: Grünecker, Kinkeldey, Stockmair & Schwanhäusser
(86) International application number: PCT/JP2012/081773
(87) International publication number: WO 2013/085026

(57) **Abstract**

Provided are a detection method and detection kit for detecting a nucleotide mutation simply, with highly sensitivity and high specificity, which allow a plurality of samples to be simultaneously detected in a single measurement. The detection method includes the steps of amplifying a nucleic acid to be tested to obtain a DNA amplification product; subjecting the DNA amplification product, a first nucleic acid probe having a region complementary to a first region of the DNA amplification product, a second nucleic acid probe having a region complementary to a second region of the DNA amplification product, and a plurality of kinds of signal amplification probes to be used in a PALSAR method to a reaction to form a complex for detection including a probe polymer; and detecting the probe polymer bound in the complex for detection to detect the presence or absence of a mutation in a nucleotide mutation site of the DNA amplification product, the first region of the DNA amplification product including the nucleotide mutation site, the nucleotide mutation site being located at a portion other than both end portions of the first region, the second nucleic acid probe having a base sequence identical to part or all of the base sequence of one of the signal amplification probes.

## Description

### Technical Field

The present invention relates to a detection method and detection kit for detecting a nucleotide mutation, and more particularly, to a detection method and detection kit for detecting nucleotide mutations such as a genetic mutation and a genetic polymorphism with high sensitivity and high specificity.

### Background Art

Individual differences in base sequences of genes are found even among organisms belonging to the same species. Of those differences in sequences, one occurring at a frequency of 1% or more is called a "genetic polymorphism" or simply a "polymorphism", and one occurring at a frequency of less than 1% is called a "genetic mutation" or simply a "mutation". The term "polymorphism" and the term "mutation" are often used with no strict distinction between their definitions. There are various kinds of polymorphisms and mutations, such as substitutions of bases, insertions and deletions of bases, and differences in the numbers of repeats of given base sequences. In addition, the numbers of bases in the substitutions, the insertions, the deletions, and the repeated sequences range from a single base to several thousand bases, in other words, a gene scale. In the present invention, those genetic polymorphisms and genetic mutations are collectively referred to as nucleotide mutations.

It should be noted that the term "gene" has a narrow sense definition referring to a region encoding an amino acid sequence for a protein (including exons and introns), and a broad sense definition referring collectively to nucleic acids including DNA and RNA transcribed therefrom. In the present invention, the term is used in the latter meaning unless otherwise specified.

In humans, those genetic polymorphisms and mutations have been applied to increasingly important technologies in medical fields such as genetic disease diagnosis (mutations in: WRN gene in Werner syndrome; RB gene, p53 gene, BRCA1 and BRCA2 genes, APC gene, and hMsh2 gene in various familial tumors; and the like), prediction of a risk of developing a disease (ß2AR, ß3AR, and UCP1 involved in obesity, and the like), prediction of effects and side effects of drugs (NAT2 gene associated with an anti-tuberculous drug isoniazid, VKORC1 and CYP2C9 genes associated with an anticoagulant warfarin, UGT1A1 gene associated with an anticancer drug irinotecan hydrochloride, and the like), and assessment of compatibility in organ transplantation, bone marrow transplantation, or the like (HLA gene).

In addition, in a disease such as cancer, acquired somatic mutations such as substitutions, insertions, and deletions in base sequences are frequently found, and are useful in diagnosis and decision of the course of therapy (p53 gene involved in various cancers, K-ras gene involved in colon cancer, pancreatic cancer, and the like, EGFR gene involved in an effect of an anticancer drug gefitinib, and the like).

In addition, the genetic polymorphisms and mutations have been applied also in the fields of forensic science and forensic medicine such as individual identification and parentage determination. Further, the genetic polymorphisms and mutations have been applied also in industrial fields essential to daily life such as cultivar identification and breeding of agricultural products, individual identification, and identification of genetically modified products.

Genetic testing technologies widely utilized in the fields of medicine and food as described above may profoundly influence human health and safety, and the environment such as an ecosystem as well. Therefore, such genetic testing technologies are required to have high accuracy and reliability above all else. In addition to the accuracy and the reliability, good efficiency of a test and an inexpensive cost for carrying out the test are also important requirements in order to achieve wide use in social welfare like medicine and daily goods like food.

Hitherto, many assessment (genotyping) methods for genetic mutations and/or genetic polymorphisms have been developed and reported. It is a sequencing method based on a Sanger method that is considered to have the highest reliability to date (Non Patent Document 1). In recent years, a cycle sequencing method in combination with a PCR method (Non Patent Document 2) and a method using an auto-sequencer have been mainstream sequencing methods, and higher speed and higher throughput thereof have been pursued. However, those methods each require an expensive apparatus or reagent, and still include many steps that require manual operation, thus being complicated. Further, accuracy of software that automatically carries out a process of converting waveform data obtained by electrophoresis into base sequence data is generally not high. Accordingly, for example, a process of confirming analysis results by human visual observation is often required. Thus, problems in terms of simplicity, rapidity, and efficiency can be pointed out.

As another method, there is known a method called a primer extension method or a minisequencing method, the method involving using a PCR product obtained by amplification from a sample to be tested as a template, incorporating one or a few nucleotides with a DNA polymerase, and identifying kinds of the incorporated nucleotides on the basis of a sequence of the template DNA, thereby carrying out genotyping (Non Patent Document 3). This method has the same basic principle as the sequencing method based on the Sanger method in that a nucleotide is incorporated from a primer with a DNA polymerase. Accordingly, the same problems can be pointed out. That is, the method involves complicated operation because the method requires purification of a PCR product prior to primer extension in order to, for example, remove a remaining PCR primer and nucleotide and exchange reaction solutions. In addition, as one of the latest methods of identifying kinds of incorporated nucleotides, a mass spectrometry method is sometimes used, but the mass spectrometry method requires a very expensive mass spectrometry apparatus (Non Patent Document 4). Thus, in the primer extension method, problems in terms of economy, simplicity, and rapidity can be pointed out.

As a simpler, rapider, and less expensive genotyping method, a hybridization-based method has been widely utilized. The hybridization-based method is mainly based on the so-called B/F separation technique, which involves: immobilizing a probe generally having a length of from about 15 to 25 bases onto a solid phase such as a microplate, a membrane, beads, or a glass slide; hybridizing a target nucleic acid with the immobilized probe to form a heteroduplex; and selectively dissociating and removing only a heteroduplex containing a mismatch during the hybridization and washing operation after the hybridization by utilizing a difference in melting temperature (hereinafter referred to as "Tm") (hereinafter referred to as "ΔTm") between the case where a mismatched base pair is present in the heteroduplex and the case where no mismatched base pair is present. The hybridization-based method has been marketed in the form of GeneChip P450, a typing reagent for cytochrome P450 (Affymetrix, Inc. /Roche) (Non Patent Document 5).

In this method, as a chain length of the probe increases, the Tm becomes higher and a ratio of the ΔTm to the Tm itself becomes lower. Accordingly, in many cases, it becomes difficult to separate a target nucleic acid containing a mismatch and a target nucleic acid containing no mismatch by adjusting washing conditions on the basis of the smaller ΔTm. In addition, as the chain length reduces, specificity of the hybridization becomes lower and the Tm also becomes lower, and hence a binding affinity between the probe and the target nucleic acid also becomes lower, which prevents stable hybridization of the probe. Accordingly, even when the B/F separation is carried out under mild conditions, a completely matched target nucleic acid and a target nucleic acid containing a mismatch are both removed owing to the lower affinity of each hybrid. As a result, in many cases, it becomes difficult to distinguish between a complete match and a mismatch.

A TaqMan PCR method involves an extension reaction step of PCR in which an oligonucleotide called a TaqMan probe hybridizes with single-stranded template DNA, a DNA polymerase continues an extension reaction from a primer, and when the DNA polymerase reaches a region in which the TaqMan probe has hybridized, the TaqMan probe is degraded from the 5' side by an 5'-3' exonuclease activity of the DNA polymerase (Non Patent Document 6). The TaqMan probe is labeled at the 5' end with a fluorophore, and at the 3' end with a quencher that absorbs fluorescence. When the TaqMan probe is not degraded, fluorescence of the labeling fluorophore is suppressed by a phenomenon known as fluorescence resonance energy transfer (FRET). However, when the TaqMan probe hybridizes with the template DNA in a sequence-specific manner and is degraded by the 5'-3' exonuclease activity of the DNA polymerase to liberate the fluorophore with which the 5' end is labeled from the TaqMan probe, fluorescence is emitted. In the TaqMan PCR method, even a single base substitution, i.e., a single nucleotide polymorphism (SNP) can be recognized. That is, when a sequence of the template DNA and a sequence of the TaqMan probe are complementary to each other, the TaqMan probe hybridizes with the template DNA, and fluorescence is emitted. However, when there is a non-complementary base pair between the TaqMan probe and the template DNA, the Tm value becomes lower than in the case of complete complementarity, and hence the TaqMan probe does not hybridize with the template DNA. However, the ΔTm is generally not large, and hence it is not easy to recognize a single base difference with high accuracy. Accordingly, in polymorphism/mutation detection by the related-art TaqMan PCR method, a problem in terms of specificity can be pointed out.

In view of the foregoing, in order to improve specificity of genotyping based on the TaqMan PCR method, a minor groove binder (MGB) probe has been devised. The MGB binds to a minor groove of a double helix structure of DNA to increase a Tm value between a TaqMan probe and a template nucleic acid, thereby stabilizing binding between the TaqMan probe and the template DNA (Non Patent Document 7). The MGB is bound to a quencher, with which the 3' end of the TaqMan probe is labeled. Thus, the Tm in the case where the TaqMan probe and the template DNA hybridize with each other in complete complementarity can be increased to increase a difference in Tm as compared to the case where a mismatch is present, i.e., ΔTm. As a result, the specificity of the genotyping based on the TaqMan PCR method is improved. Performance of SNP typing based on the TaqMan method using the MGB probe is described in Non Patent Document 8.

In general, however, in the TaqMan PCR method, the number of kinds of genetic polymorphism/mutation markers that can be assessed in one reaction container is only 1, and it is difficult to simultaneously assess a plurality of genetic polymorphisms/mutations in a single reaction container. Accordingly, the method cannot be efficiently used in, for example, HLA typing, cultivar identification, individual identification, diagnosis of diseases including cancer, genetic disease diagnosis, a drug sensitivity prediction test, and a side effect development prediction test for a drug, each of which requires typing of a plurality of kinds of genetic markers.

Further, in the TaqMan PCR method, a ratio of a base sequence-specific signal to a background signal, i.e., an S/N ratio is low, and hence it is necessary to carry out cluster analysis involving simultaneously assaying a plurality (generally several tens) of samples, and performing genotyping on the basis of a distribution of their signals. A genotyping method that requires cluster analysis requires simultaneous assay of a plurality of control samples even in the case of performing typing of a small number of samples. This is considered to cause significant problems in terms of cost for carrying out a test and operating efficiency.

A detection method for a genetic polymorphism/mutation called an Invader method (Non Patent Document 9) uses an oligonucleotide probe specific to a target gene, and has a feature in that the method is not based on a principle that utilizes ΔTm. That is, the Invader method uses an endonuclease called Cleavase, which, when two kinds of probes specifically hybridize with a target nucleic acid, recognizes a structure of a complex formed by the target nucleic acid and the two kinds of probes and cleaves one of the probes. One of the probes is labeled with a fluorophore and a quencher, and only when this probe is cleaved by Cleavase, a fluorescence signal is emitted. On the other hand, when a difference is present in a base sequence of the target nucleic acid, a difference occurs in the structure of the complex formed with the two kinds of probes. In this case, Cleavase, which is a structure-specific endonuclease, does not cleave the probe, and hence fluorescence is not emitted. Such presence or absence of an increase in fluorescence signal allows a base difference to be recognized.

However, the Invader method is not a gene amplification method, and hence requires a reaction step of several hours in order to obtain a base sequence-specific signal. In addition, as in the TaqMan PCR method, the number of kinds of polymorphism/mutation markers that can be assessed in one reaction container is limited, and is 1 in many cases. Further, also in the Invader method, because of its low S/N ratio, genotyping often requires cluster analysis.

In addition, Patent Document 1 discloses a detection method for a mutation gene involving utilizing a PALSAR method, which involves forming a high molecular structure from oligonucleotides and can be utilized as a signal amplification method, and a ligation reaction with a DNA ligase. The PALSAR method is a signal amplification technology in which a plurality of kinds of signal amplification probes having complementary base sequence regions capable of hybridizing with each other and being capable of forming a probe polymer through a self-assembly reaction form a high molecular structure through a self-assembly reaction while recognizing each other, and the method has a feature in that a signal is amplified under isothermal conditions without requiring an enzymatic reaction. In Patent Document 1, there is adopted such a configuration that when a first probe having a region complementary to a signal amplification probe and a capture probe are allowed to hybridize with target DNA, an end of the capture probe is located at a nucleotide mutation site of the target DNA, and the capture probe and the first probe are annealed to the target DNA so that the end and the first probe may be adjacent to each other. Only when the nucleotide mutation site of the target DNA and the capture probe are complementary to each other, the capture probe and the first probe adjacent to each other are ligated by an action of the ligase, and the presence of the ligated probe is subjected to signal amplification by the PALSAR method to enable detection of a specific sequence with high sensitivity while distinguishing even a single base difference.

Further, Patent Document 2 discloses an assist probe to be used in the PALSAR method and a method of forming a signal probe polymer using the assist probe. A target gene can be detected by forming a signal probe polymer through the use of an assist probe having a target region capable of hybridizing with the target gene in the method of Patent Document 1.

However, the methods described in Patent Document 1 and Patent Document 2 depend on a DNA-binding activity of a ligase, which depends on complementarity between a probe and a target nucleic acid. The PALSAR method improves detection sensitivity of those methods, and detection can be sufficiently carried out by the methods. However, specificity of genetic mutation detection in those methods depends on that of the ligase. That is, when a ligase having low specificity is used in the method described in Patent Document 1, there may cause a problem in that a non-specific signal is amplified by the PALSAR method. In addition, combining the PALSAR method, which has a feature in basically requiring no enzymatic reaction, with a method requiring an enzymatic reaction gives rise to another problem in that operation is complicated to some degree.

Meanwhile, as a technology involving forming a high-order structure with nucleic acids such as DNA, for use in a signal amplification method for nucleic acid detection, there are known a branched DNA method and a dendrimer method (Non Patent Document 10). Those technologies have already been put into industrial use for the purpose of detecting microorganism-derived DNA or RNA without performing gene amplification. However, it has not been discussed how those signal amplification technologies may be usefully utilized in detection of genetic polymorphisms and mutations.

### Prior Art Documents

### Patent Documents

[Patent Document 1] WO 2004/074480 A1
[Patent Document 2] WO 2006/093097 A1

### Non Patent Documents

[Non Patent Document 1] Sambrook et al. Molecular Cloning, 1898; Second Edition: Chapter 13
[Non Patent Document 2] Gyllensten et al. Biotechniques 1989; 7: 700-708
[Non Patent Document 3] Syvanen et al. Genomics 1990; 9: 119-127
[Non Patent Document 4] Haff et al. Genome Research 1997; 7: 378-388
[Non Patent Document 5] Jain et al. Molecular Diagnostics 2005; 9: 119-127
[Non Patent Document 6] Holland et al. Proceedings of the National Academy of Sciences of the United States of America 1991; 88: 7276-7280
[Non Patent Document 7] Kutyavin et al. Nucleic Acids Research 2000; 28: 655-661
[Non Patent Document 8] Dela Vega et al. Mutation Research 2005; 573: 111-135
[Non Patent Document 9] Oliver et al. Mutation Research 2005; 573: 103-110
[Non Patent Document 10] Lowe et al. Cytometry Part A 2004; 60A: 135-144

### Disclosure of the Invention

### Problem to be solved by the Invention

In view of the problems of the related art described above, an object of the present invention is to provide a detection method and detection kit for detecting a nucleotide mutation simply, with highly sensitivity and high specificity, which allow a plurality of samples to be simultaneously detected in a single measurement.

### Means for solving the Problem

The inventors of the present invention have made extensive studies, and as a result, have found that by using: a first nucleic acid probe having a region complementary to a nucleotide mutation site-containing first region of a nucleic acid to be tested; a signal amplification probe; and a second nucleic acid probe having a region complementary to the signal amplification probe and a region complementary to the first region of the nucleic acid to be tested, and by adopting such a configuration that the nucleotide mutation site of the nucleic acid to be tested is located at a portion other than both ends of the first region, that is, the nucleotide mutation site of the nucleic acid to be tested is not to be located at an end of the first nucleic acid probe when the nucleic acid to be tested and the first nucleic acid probe are subjected to a reaction, the presence or absence of a mutation in a nucleotide mutation site of a DNA amplification product obtained by amplification from the nucleic acid to be tested can be detected without requiring a ligation reaction with an enzyme.

That is, a detection method for a nucleotide mutation of the present invention includes the steps of: (a) amplifying a nucleic acid to be tested through an amplification reaction to obtain a DNA amplification product; (b) subjecting the DNA amplification product, a first nucleic acid probe having a first target sequence region complementary to a first region of the DNA amplification product, a second nucleic acid probe having a second target sequence region complementary to a second region of the DNA amplification product, and a plurality of kinds of signal amplification probes having complementary base sequence regions capable of hybridizing with each other and being capable of forming a probe polymer through a self-assembly reaction to a reaction to form a complex for detection including the DNA amplification product, the first nucleic acid probe, the second nucleic acid probe, and the probe polymer formed from the plurality of kinds of signal amplification probes; and (c) detecting the probe polymer bound in the complex for detection to detect the presence or absence of a mutation in a nucleotide mutation site of the DNA amplification product, the first region of the DNA amplification product including the nucleotide mutation site, the nucleotide mutation site being located at a portion other than both end portions of the first region of the DNA amplification product, the second nucleic acid probe having a base sequence identical to part or all of a base sequence of one of the plurality of kinds of signal amplification probes.

A detection kit of the present invention is a detection kit to be used for detection of a nucleotide mutation in a DNA amplification product obtained by amplification through a polymerase chain reaction, the detection kit including: a first nucleic acid probe; a second nucleic acid probe; and a plurality of kinds of signal amplification probes having complementary base sequence regions capable of hybridizing with each other and being capable of forming a probe polymer through a self-assembly reaction, in which: the first nucleic acid probe has a first target sequence region complementary to a first region of the DNA amplification product; the second nucleic acid probe has a second target sequence region complementary to a second region of the DNA amplification product; the first region of the DNA amplification product includes a nucleotide mutation site, the nucleotide mutation site being located at a portion other than both end portions of the first region of the DNA amplification product; and the second nucleic acid probe has a base sequence identical to part or all of a base sequence of one of the plurality of kinds of signal amplification probes.

In each of the detection method for a nucleotide mutation of the present invention and the detection kit of the present invention, the first region and second region of the DNA amplification product may be adjacent to each other, or may not be adjacent to each other.

In each of the detection method for a nucleotide mutation of the present invention and the detection kit of the present invention, it is preferred that: the first nucleic acid probe have the first target sequence region at the 5' end or the 3' end; and the second nucleic acid probe have the second target sequence region at the 3' end or the 5' end.

In each of the detection method for a nucleotide mutation of the present invention and the detection kit of the present invention, it is preferred that the first nucleic acid probe be immobilized onto a support or have introduced therein a moiety capable of being immobilized onto a support.

In each of the detection method for a nucleotide mutation of the present invention and the detection kit of the present invention, it is preferred that at least one of the plurality of kinds of signal amplification probes be labeled with a labeling substance.

In each of the detection method for a nucleotide mutation of the present invention and the detection kit of the present invention, as a first example, it is preferred that: the plurality of kinds of signal amplification probes include a first signal amplification probe and a second signal amplification probe; the first signal amplification probe include a nucleic acid probe including three or more nucleic acid regions that include at least a nucleic acid region X, a nucleic acid region Y, and a nucleic acid region Z in the stated order from the 5' end side; and the second signal amplification probe include a nucleic acid probe including three or more nucleic acid regions that include at least a nucleic acid region X' complementary to the nucleic acid region X, a nucleic acid region Y' complementary to the nucleic acid region Y, and a nucleic acid region Z' complementary to the nucleic acid region Z in the stated order from the 5' end side.

In each of the detection method for a nucleotide mutation of the present invention and the detection kit of the present invention, as a second example and a third example, it is preferred that: the plurality of kinds of signal amplification probes include a plurality of dimer probes or dimer formation probes for forming the dimer probes; each of the plurality of dimer probes include a dimer formed from two kinds of dimer formation probes; each of the dimer formation probes include three regions, i.e., a 5' side region, a mid-region, and a 3' side region; in the two kinds of dimer formation probes for forming the dimer, the mid-regions be complementary to each other and the 3' side regions and the 5' side regions be non-complementary to each other; and each of the 5' side regions of each of the plurality of dimer probes be complementary to any one of the 5' side regions of other dimer probes and each of the 3' side regions of each of the dimer probes be complementary to any one of the 3' side regions of the other dimer probes.

In each of the detection method for a nucleotide mutation of the present invention and the detection kit of the present invention, as a fourth example and a fifth example, it is preferred that: the plurality of kinds of signal amplification probes include one or more pairs of dimer formation probes or one or more dimer probes to be formed from the pairs of dimer formation probes, and one or more kinds of cross-linking probes; each of the dimer formation probes include three regions, i.e., a 5' side region, a mid-region, and a 3' side region, the mid-regions of each of the pairs of dimer formation probes be complementary to each other, all of the 3' side regions and 5' side regions of each of the pairs of dimer formation probes be non-complementary to each other; each of the cross-linking probes include two regions, i.e., a 5' side region and a 3' side region; the 5' side region of each of the dimer formation probes be complementary to any one of the 5' side regions of the cross-linking probes, and each of the 3' side regions of the dimer formation probes be complementary to any one of the 3' side regions of the cross-linking probes.

### Advantageous Effects of Invention

According to one embodiment of the present invention, it is possible to provide the simple, highly sensitive, and highly specific detection method for a nucleotide mutation and detection kit requiring no enzymatic reaction with an enzyme such as a ligase. In addition, when the present invention is combined with a conventional platform technology in genetic testing such as a DNA chip, microarray, or bead array technology, a large number of nucleotide mutations can be simultaneously detected with high sensitivity and high specificity.

### Brief Description of the Drawings

FIGS. 1 are schematic explanatory diagrams illustrating an example of a detection method for a nucleotide mutation of the present invention, FIG. 1(a) is a schematic explanatory diagram of Step (b1) in the case where no mutation is present in a nucleotide mutation site of a DNA amplification product, and FIG. 1(b) is a schematic explanatory diagram of Step (b1) in the case where a mutation is present in the nucleotide mutation site of the DNA amplification product.
FIGS. 2 are schematic explanatory diagrams illustrating an example of the detection method for a nucleotide mutation of the present invention, FIG. 2(a) is a schematic explanatory diagram of Step (b2) in the case where no mutation is present in the nucleotide mutation site of the DNA amplification product, and FIGS. 2(b) and 2(c) are schematic explanatory diagrams of Step (b2) in the case where a mutation is present in the nucleotide mutation site of the DNA amplification product.
FIGS. 3 are schematic explanatory diagrams illustrating another example of the detection method for a nucleotide mutation of the present invention, FIG. 3(a) is a schematic explanatory diagram of Step (b1) in the case where no mutation is present in the nucleotide mutation site of the DNA amplification product, and FIG. 3(b) is a schematic explanatory diagram of Step (b1) in the case where a mutation is present in the nucleotide mutation site of the DNA amplification product.
FIG. 4 is a schematic explanatory diagram illustrating a first example of a plurality of kinds of signal amplification probes to be used in probe polymer formation.
FIG. 5 is a schematic explanatory diagram illustrating a probe polymer to be formed using the plurality of kinds of signal amplification probes illustrated in FIG. 4.
FIGS. 6 are schematic explanatory diagrams illustrating a second example of the plurality of kinds of signal amplification probes to be used in probe polymer formation.
FIG. 7 is a schematic explanatory diagram illustrating a probe polymer to be formed using the plurality of kinds of signal amplification probes illustrated in FIGS. 6.
FIG. 8 is a schematic explanatory diagram illustrating another example of a second dimer probe in the plurality of kinds of signal amplification probes illustrated in FIGS. 6.
FIGS. 9 are schematic explanatory diagrams illustrating a third example of the plurality of kinds of signal amplification probes to be used in probe polymer formation.
FIGS. 10 are schematic explanatory diagrams illustrating a forth example of the plurality of kinds of signal amplification probes to be used in probe polymer formation.
FIG. 11 is a schematic explanatory diagram illustrating a probe polymer to be formed using the plurality of kinds of signal amplification probes illustrated in FIGS. 10.
FIGS. 12 are schematic explanatory diagrams illustrating two dimer probes in a fifth example of the plurality of kinds of signal amplification probes to be used in probe polymer formation.
FIG. 13 is a schematic explanatory diagram illustrating another example of cross-linking probes in the plurality of kinds of signal amplification probes illustrated in FIGS. 12.
FIG. 14 is a schematic explanatory diagram illustrating a probe polymer to be formed using the dimer probe illustrated in FIGS. 12 and the cross-linking probes illustrated in FIG. 13.
FIGS. 15 are schematic explanatory diagrams illustrating two pairs of cross-linking probes in the fifth example of the plurality of kinds of signal amplification probes to be used in probe polymer formation.
FIGS. 16 are schematic explanatory diagrams illustrating a sixth example of the plurality of kinds of signal amplification probes to be used in probe polymer formation.
FIG. 17 is a schematic explanatory diagram illustrating a probe polymer to be formed using the plurality of kinds of signal amplification probes illustrated in FIGS. 16.
FIG. 18 is a schematic explanatory diagram illustrating a probe polymer to be formed using the plurality of kinds of signal amplification probes illustrated in FIGS. 12 and FIGS. 15.
FIG. 19 is a graph showing the results of Examples 1 to 3.
FIG. 20 is a graph showing the results of Example 4.

### Best Mode for carrying out the Invention

Embodiments of the present invention are described below with reference to the accompanying drawings. It should be appreciated that the illustrated examples are given for illustrative purposes only and various modifications may be made without departing from the technical concept of the present invention.

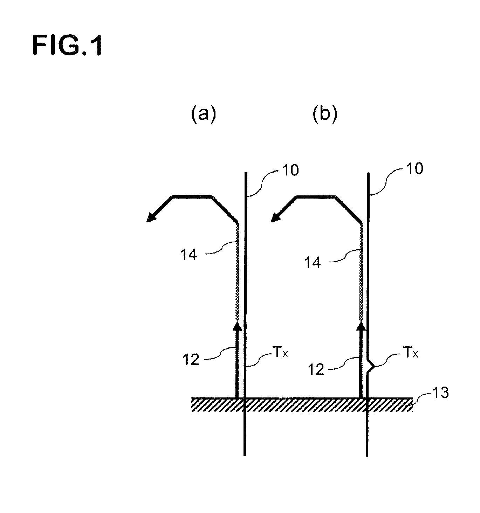

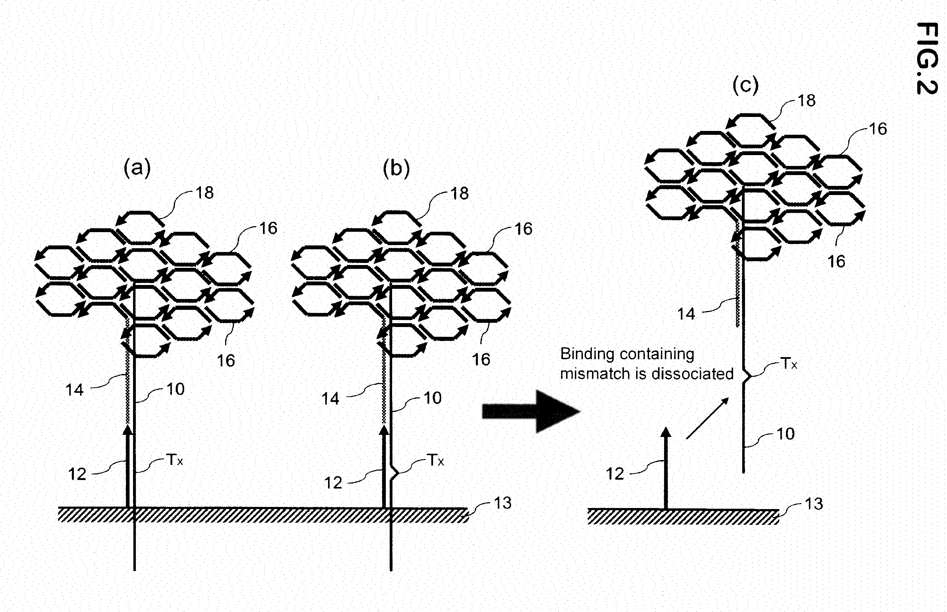

FIGS. 1 and 2 are schematic explanatory diagrams illustrating an example of a detection method for a nucleotide mutation of the present invention. FIG. 1(a) is a schematic explanatory diagram of Step (b1) in the case where no mutation is present in a nucleotide mutation site of a DNA amplification product, and FIG. 1(b) is a schematic explanatory diagram of Step (b1) in the case where a mutation is present in the nucleotide mutation site of the DNA amplification product. FIG. 2(a) is a schematic explanatory diagram of Step (b2) in the case where no mutation is present in the nucleotide mutation site of the DNA amplification product, and FIGS. 2(b) and 2(c) are schematic explanatory diagrams of Step (b2) in the case where a mutation is present in the nucleotide mutation site of the DNA amplification product.

In FIGS. 1 and FIGS. 2, reference symbol 10 denotes a DNA amplification product obtained in Step (a), and reference symbol Tx denotes a nucleotide mutation site. Reference symbol 12 denotes a first nucleic acid probe (hereinafter referred to as capture probe), reference symbol 14 denotes a second nucleic acid probe (hereinafter referred to as assist probe), and reference symbol 16 denotes a signal amplification probe.

A detection kit of the present invention is a detection kit to be used for detection of a nucleotide mutation in a DNA amplification product obtained by amplification through a polymerase chain reaction, and includes a capture probe 12, an assist probe 14, and a plurality of kinds of signal amplification probes 16 having complementary base sequence regions capable of hybridizing with each other and being capable of forming a probe polymer through a self-assembly reaction.

In the present invention, complementary base sequences may be any base sequences that can hybridize with each other. In addition, non-complementary base sequences may be any base sequences that do not hybridize with each other. The non-complementary base sequences also encompass base sequences that are identical to each other.

The capture probe 12 is a nucleic acid probe having a first target sequence region complementary to a first region of a DNA amplification product 10, and the first region of the DNA amplification product 10 includes a nucleotide mutation site Tx at a portion other than both end portions of the first region. That is, as illustrated in FIGS. 1, there is adopted such a configuration that when the DNA amplification product 10 and the capture probe 12 hybridize with each other, the nucleotide mutation site Tx of the DNA amplification product 10 is not to be located at an end of the capture probe 12. When the nucleotide mutation site Tx is located near any one of the ends of the capture probe 12, the sensitivity or specificity of detection, or both of them may deteriorate. The capture probe 12 is desirably designed so that the nucleotide mutation site Tx to be detected is located near its mid-portion. Specifically, the nucleotide mutation site Tx is preferably located away from each of both ends of the capture probe 12 by 1 base or more, more preferably 3 bases or more, still more preferably 5 bases or more.

In addition, the first target sequence region of the capture probe 12 may be any region of the probe, but is preferably located away from an end of the capture probe 12 by 5 bases or less, is more preferably located away from the end of the capture probe 12 by 3 bases or less, and is most preferably located at the end of the capture probe 12.

In ordinary cases, as the capture probe, there may be used an oligonucleotide probe chemically synthesized by a known method such as an amidite method. In addition to DNA and RNA, a chimeric molecule of DNA and RNA, or an artificial nucleotide such as PNA or LNA may also be used. The chain length of the capture probe, which varies depending on reaction conditions, is from about 7 to 35 bases, more preferably from about 15 to 25 bases. Such chemically synthesized oligonucleotide is commercially available.

It is suitable that the capture probe 12 be immobilized onto a support 13 or have introduced therein a moiety capable of being immobilized onto the support. When the support is used in a detection system, the capture probe is used by allowing one of its ends to directly or indirectly bind to the support. As the support, there may be used one formed of any material and having any shape to be used in, for example, a related-art gene analysis technology or enzyme immunoassay, such as a substrate made of a synthetic resin or glass to be used for a microplate, a membrane filter, a DNA chip, or a microarray, or fine particles to be used for a bead array.

It should be noted that in FIGS. 1 to 3, there is illustrated an example in which the 5' end of the capture probe 12 is allowed to bind to the support 13, but in the present invention, a site of the capture probe 12 to be allowed to bind to the support 13 is not particularly limited. The site of the capture probe 12 to be allowed to bind to the support 13 may be an end of the capture probe, or may be an inner region other than the end. Of those, a 5' or 3' end is preferably utilized.

The nucleic acid probe is allowed to bind to the support by a method based on a chemical bond, a method based on a biological interaction, a method based on physical adsorption, or the like. In the method based on a chemical bond, for example, in the case of using a support coated with a carboxyl group, a coupling reaction between the carboxyl group and an amino group with which an oligonucleotide is labeled can be allowed to occur. In the method based on a biological interaction, for example, a binding force between streptavidin with which the support is coated and biotin with which the nucleic acid probe is modified can be utilized. In addition, in the method based on physical adsorption, for example, in the case of using a solid phase having negative charge, an oligonucleotide labeled with a substance having positive charge such as an amino group can be allowed to electrostatically adsorb onto the support.

The assist probe 14 is a nucleic acid probe having a second target sequence region complementary to a second region of the DNA amplification product 10, and having a base sequence identical to part or all of the base sequence of one of the plurality of kinds of signal amplification probes 16.

The second target sequence region of the assist probe 14 is preferably located at an end of the assist probe 14. Specifically, when the capture probe 12 has the first target sequence region at its 5' end, the assist probe 14 preferably has the second target sequence region at its 3' end, and when the capture probe 12 has the first target sequence region at its 3' end, the assist probe 14 preferably has the second target sequence region at its 5' end.

It should be noted that in FIGS. 1 and 2, there is illustrated an example of such a configuration that: the first region and second region of the DNA amplification product 10 are adjacent to each other; and the capture probe 12 and the assist probe 14, in a state of being adjacent to each other, hybridize with the DNA amplification product 10, but in the present invention, the first region and second region of the DNA amplification product 10 may be adjacent to each other, or may not be adjacent to each other, without any particular limitation.

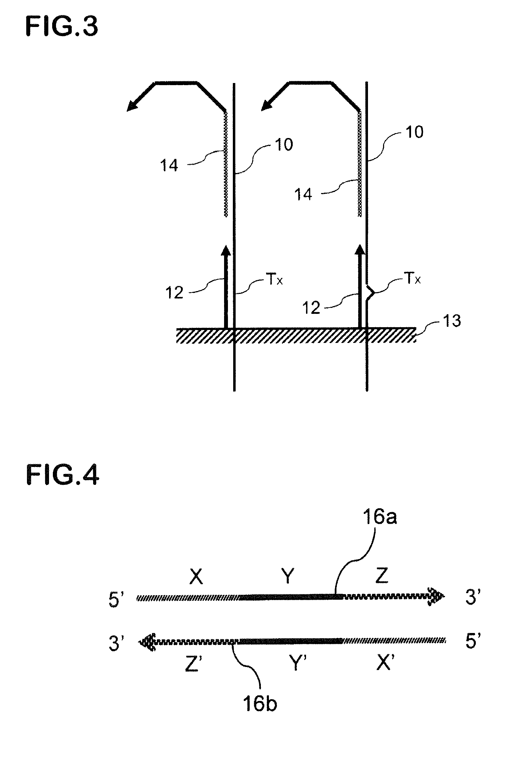

FIGS. 3 are schematic explanatory diagrams illustrating another example of the detection method for a nucleotide mutation of the present invention. As illustrated in FIGS. 3, there may be adopted such a configuration that: the first region and second region of the DNA amplification product 10 are not adjacent to each other; and the capture probe 12 and the assist probe 14, in a state of being located away from each other by 1 base or more, hybridize with the DNA amplification product 10. It is preferred to adopt such a configuration that the capture probe 12 and the assist probe 14, in a state of being located away from each other by from 0 to 20 bases, hybridize with the DNA amplification product 10, and a range of from 0 to 3 bases is more preferred. It should be noted that a state in which the capture probe 12 and the assist probe 14 are located away from each other by 0 bases is a state illustrated in FIGS. 1.

In addition, in FIGS. 1 to 3, there is illustrated an example in which the assist probe has a base sequence identical to all of the base sequence of one signal amplification probe (sequence XYZ). In the present invention, however, a region having a base sequence identical to that of one of the signal amplification probes and being capable of binding to the other signal amplification probes is not particularly limited as long as the sequence enables specific binding between the assist probe and the signal amplification probes, and a sequence having a base sequence identical to part of the base sequence of one of the signal amplification probes (e.g., sequence X, sequence Y, sequence Z, sequence XY, sequence YZ, sequence ZYZ, sequence XYX, or partial sequences of these sequences) may be used.

In ordinary cases, as the assist probe as well, there may be utilized an oligonucleotide probe chemically synthesized by an amidite method or the like. In addition to DNA and RNA, a chimeric molecule of DNA and RNA, and an artificial nucleotide such as PNA or LNA may also be used. Although the chain length of the assist probe varies depending on reaction conditions, it is suitable that the length of the second target sequence region be preferably from about 7 to 35 bases, more preferably from about 12 to 25 bases. Thus, the full length of the assist probe is a length obtained by adding thereto a sequence complementary to part or all of the signal amplification probe.

The plurality of kinds of signal amplification probes 16 are nucleic acid probes having complementary base sequence regions capable of hybridizing with each other and being capable of forming a probe polymer through a self-assembly reaction. In FIGS. 1 to 3, there is illustrated an example in the case of using, as the plurality of kinds of signal amplification probes 16, a first signal amplification probe (HCP-1) 16a, which includes a nucleic acid region X, a nucleic acid region Y, and a nucleic acid region Z in the stated order from its 5' end portion, and a second signal amplification probe (HCP-2) 16b, which includes a nucleic acid region X' complementary to the nucleic acid region X, a nucleic acid region Y' complementary to the nucleic acid region Y, and a nucleic acid region Z' complementary to the nucleic acid region Z in the stated order from its 5' end portion.

In FIGS. 1 to 3, there is illustrated an example of using, as the plurality of kinds of signal amplification probes 16, the first signal amplification probe 16a including three nucleic acid regions and the second signal amplification probe 16b including three nucleic acid regions, illustrated in FIG. 4 to be described later. However, the plurality of kinds of signal amplification probes to be used in the method of the present invention are not particularly limited as long as the probes have complementary base sequence regions capable of hybridizing with each other and are capable of forming a probe polymer through a self-assembly reaction. Specific examples of the plurality of kinds of signal amplification probes to be used in the method of the present invention include three aspects (PALSAR I, PALSAR II, and PALSAR III) to be described later. It should be noted that the three aspects (PALSAR I, PALSAR II, and PALSAR III) are collectively referred to as PALSAR method.

### (PALSAR I)

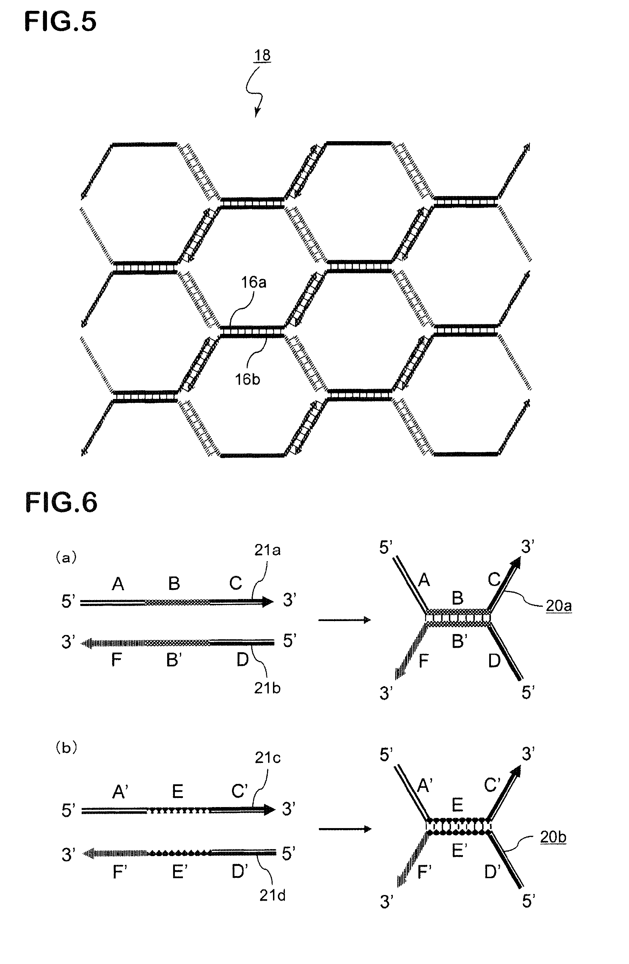

FIG. 4 is a schematic explanatory diagram illustrating a first example of the plurality of kinds of signal amplification probes to be used in probe polymer formation. FIG. 5 is a schematic explanatory diagram illustrating a polymer to be formed using the plurality of kinds of signal amplification probes illustrated in FIG. 4.

Examples of the plurality of kinds of signal amplification probes to be used in probe polymer formation include, as illustrated in FIG. 4, two kinds of signal amplification probes, i.e., the first signal amplification probe 16a having at least n (n≥3) nucleic acid regions, i.e., a nucleic acid region X₁, a nucleic acid region X₂, ·· and a nucleic acid region Xₙ in the stated order from the 5' end side, and the second signal amplification probe 16b having at least n (n≥3) nucleic acid regions, i.e., a nucleic acid region X₁' complementary to the nucleic acid region X₁, a nucleic acid region X₂' complementary to the nucleic acid region X₂, ·· and a nucleic acid region Xₙ' complementary to the nucleic acid region Xₙ in the stated order from the 5' end side. It should be noted that in FIG. 4, there is illustrated an example of a case where n represents 3. A number represented by n is not particularly limited as long as the number is 3 or more, but is preferably 3. In FIG. 4, when n represents 3, X₂ is Y and X₃ is Z.

As illustrated in FIG. 5, by allowing the two kinds of signal amplification probes 16a and 16b to hybridize with each other, the two kinds of signal amplification probes 16a and 16b self-assemble to form a polymer 18 of nucleic acid probes.

### (PALSAR II)

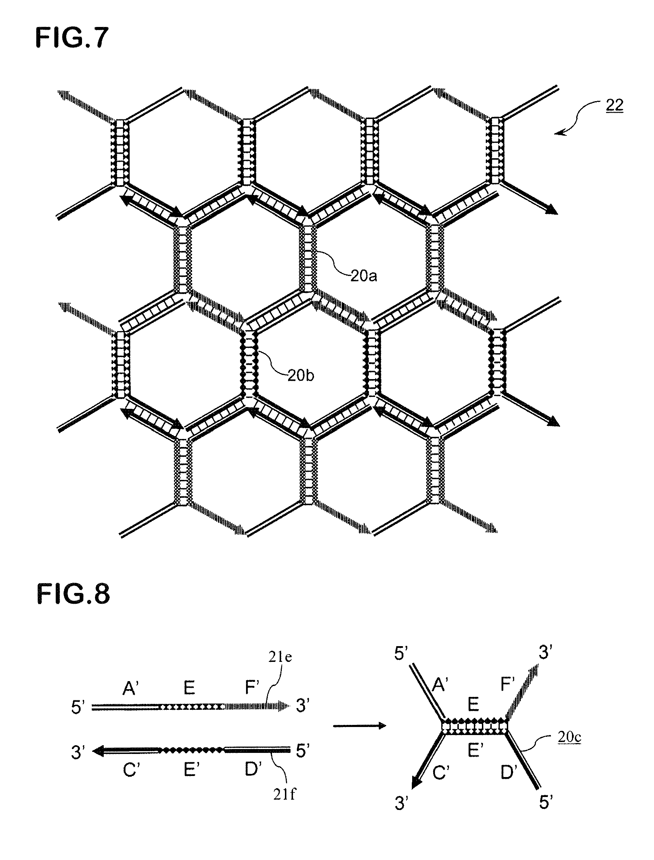

A second example of the plurality of kinds of signal amplification probes to be used in probe polymer formation can be described with reference to FIGS. 6. FIG. 7 is a schematic explanatory diagram illustrating a probe polymer to be formed using the plurality of signal amplification probes illustrated in FIGS. 6.

Examples of the plurality of kinds of signal amplification probes to be used in probe polymer formation include, as illustrated in FIGS. 6, a plurality of dimer probes 20a and 20b for forming a probe polymer, and dimer formation probes for forming the dimer probes. The plurality of dimer probes are configured so that: each of the 5' side regions of one of the plurality of dimer probes is complementary to any one of the 5' side regions of the other dimer probes; and each of the 3' side regions of one of the plurality of dimer probes is complementary to any one of the 3' side regions of the other dimer probes. Thus, the plurality of dimer probes can self-assemble in themselves to form an assembly (probe polymer). For example, nucleic acid probes disclosed in WO 02/31192 A1 are used.

In FIGS. 6, there is illustrated an example of a case of using two dimer probes (the first dimer probe 20a and the second dimer probe 20b).

As illustrated in FIG. 6(a), the first dimer probe 20a is formed by allowing two kinds of single-stranded nucleic acid probes (a first dimer formation probe 21a and a second dimer formation probe 21b) to hybridize with each other. The first dimer formation probe 21a includes three regions, i.e., a 5' side region (region A), a mid-region (region B), and a 3' side region (region C), and the second dimer formation probe 21b includes three regions, i.e., a 5' side region (region D), a mid-region (region B'), and a 3' side region (region F). In the first dimer formation probe 21a and the second dimer formation probe 21b, the mid-regions (regions B and B') are complementary to each other, and the 3' side regions (regions C and F) and the 5' side regions (regions A and D) are non-complementary to each other.

As illustrated in FIG. 6(b), the second dimer probe 20b is formed by allowing two kinds of single-stranded nucleic acid probes (a third dimer formation probe 21c and a fourth dimer formation probe 21d) to hybridize with each other. The third dimer formation probe 21c includes three regions, i.e., a 5' side region (region A'), a mid-region (region E), and a 3' side region (region C'), and the fourth dimer formation probe 21d includes three regions, i.e., a 5' side region (region D'), a mid-region (region E'), and a 3' side region (region F'). In the third dimer formation probe 21c and the fourth dimer formation probe 21d, the mid-regions (regions E and E') are complementary to each other, and the 3' side regions (regions C' and F') and the 5' side regions (regions A' and D') are non-complementary to each other.

It should be noted that in the present invention, the region A' means a region having a base sequence complementary to the region A, the region C' means a region having a base sequence complementary to the region C, the region D' means a region having a base sequence complementary to the region D, and the region F' means a region having a base sequence complementary to the region F.

The 5' side regions (regions A and D) of the first dimer probe 20a are complementary to the 5' side regions (regions A' and D') of the second dimer probe 20b, and the 3' side regions (regions C and F) of the first dimer probe 20a are complementary to the 3' side regions (regions C' and F') of the second dimer probe 20b. Thus, the first and second dimer probes 20a and 20b can be allowed to hybridize with each other to form a probe polymer 22 of nucleic acid probes (FIG. 7).

In the present invention, the dimer formation probes before dimer probe formation (21a and 21b, and 21c and 21d in FIGS. 6) may be used in place of the dimer probes, but the dimer probes are preferably used.

In FIGS. 6, there is illustrated an example in which: the 5' side region and 3' side region of the first dimer formation probe 21a are complementary to the 5' side region and 3' side region of the third dimer formation probe 21c, respectively; and the 5' side region and 3' side region of the second dimer formation probe 21b are complementary to the 5' side region and 3' side region of the fourth dimer formation probe 21d, respectively. In the present invention, however, the dimer probes only need to be configured so that: the 5' side region of one dimer probe is complementary to the 5' side region of another dimer probe; and the 3' side region of one dimer probe is complementary to the 3' side region of another dimer probe.

FIG. 8 is a schematic explanatory diagram illustrating another example of the second dimer probe to be used together with the first dimer probe 20a illustrated in FIGS. 6.

As illustrated in FIG. 8, a dimer probe 20c formed by allowing a dimer formation probe 21e, whose 5' side region is a region (region A') complementary to the 5' side region of the first dimer formation probe 21a and whose 3' side region is a region (region F') complementary to the 3' side region of the second dimer formation probe 21b, and a dimer formation probe 21f, whose 5' side region is a region (region D') complementary to the 5' side region of the second dimer formation probe 21b and whose 3' side region is a region (region C') complementary to the 3' side region of the first dimer formation probe 21a, to hybridize with each other may also be used as the second dimer probe.

In addition, in FIGS. 6, there is illustrated an example of using two kinds of dimer probes, but more kinds of dimer probes may also be used by devising a positional relationship between complementary regions (WO 02/31192 A1).

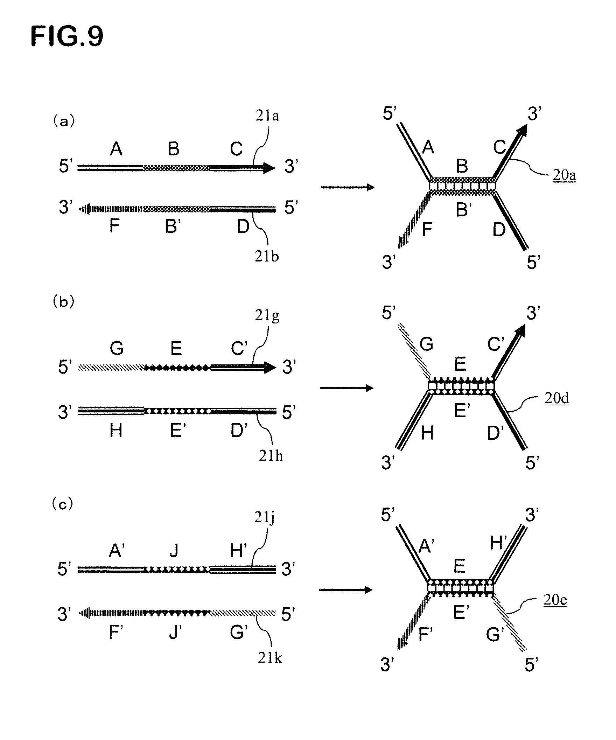

FIGS. 9 are schematic explanatory diagrams illustrating a third example of the plurality of kinds of signal amplification probes to be used in probe polymer formation, and are schematic explanatory diagrams illustrating an example of a case of using three dimer probes (the first dimer probe 20a, a second dimer probe 20d, a third dimer probe 20e).

In FIG. 9(a), the first dimer probe 20a is configured in the same manner as in FIG. 6(a).

In FIG. 9(b), the second dimer probe 20d is formed by allowing two kinds of single-stranded nucleic acid probes (dimer formation probes 21g, 21h) to hybridize with each other. The dimer formation probe 21g includes three regions, i.e., a 5' side region (region G), a mid-region (region E), and a 3' side region (region C'), in which the 3' side region (region C') is complementary to the 3' side region (region C) of the first dimer probe 20a. The dimer formation probe 21h includes three regions, i.e., a 5' side region (region D'), a mid-region (region E'), and a 3' side region (region H), in which the 5' side region (region D') is complementary to the 5' side region (region D) of the first dimer probe 20a.

In FIG. 9(c), the third dimer probe 20e is formed by allowing two kinds of single-stranded nucleic acid probes (dimer formation probes 21j, 21k) to hybridize with each other. The dimer formation probe 21j includes three regions, i.e., a 5' side region (region A'), a mid-region (region J), and a 3' side region (region H'), in which: the 5' side region (region A') is complementary to the 5' side region (region A) of the first dimer probe 20a; and the 3' side region (region H') is complementary to the 3' side region (region H) of the second dimer probe 20d. The dimer formation probe 21k includes three regions, i.e., a 5' side region (region G'), a mid-region (region J'), and a 3' side region (region F'), in which: the 5' side region (region G') is complementary to the 5' side region (region G) of the second dimer probe 20d; and the 3' side region (region F') is complementary to the 3' side region (region F) of the first dimer probe 20a. It should be noted that in FIG. 9(c), the region J' is a region complementary to the region J.

That is, in FIGS. 9, there is adopted such a configuration that: one 5' side region and one 3' side region of the first dimer probe are complementary to one 5' side region and one 3' side region of the second dimer probe, respectively; the other 5' side region and the other 3' side region of the first dimer probe are complementary to one 5' side region and one 3' side region of the third dimer probe, respectively; and the other 5' side region and the other 3' side region of the second dimer probe are complementary to the other 5' side region and the other 3' side region of the third dimer probe, respectively.

As illustrated in FIGS. 9, a probe polymer as an assembly of dimer probes is formed by: using a plurality of kinds of dimer probes configured so that each of the 3' side regions of each of the dimer probes is complementary to any one of the 3' side regions of the other dimer probes, and each of the 5' side regions of each of the dimer probes is complementary to any one of the 5' side regions of the other dimer probes; and allowing these plurality of kinds of dimer probes to hybridize with each other.

It should be noted that in the present invention, a combination of dimer probes having a complementary relationship is not particularly limited, but as illustrated in FIGS. 9, it is preferred to adopt such a configuration that one 3' side region and one 5' side region in each of the dimer probes are complementary to one 3' side region and one 5' side region in any one of the other dimer probes, respectively.

### (PALSAR III)

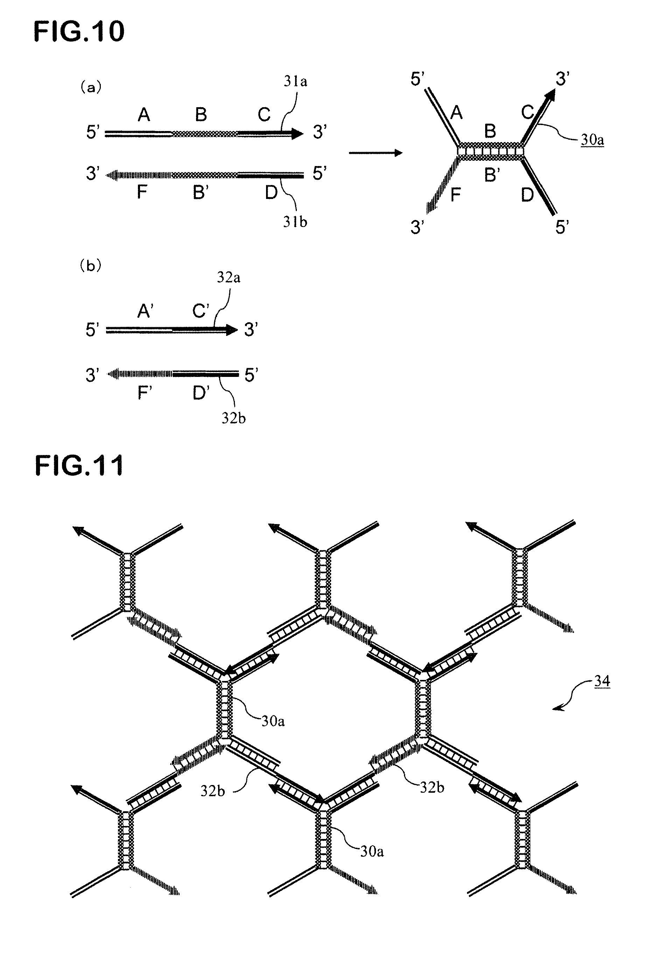

FIGS. 10 are schematic explanatory diagrams illustrating a fourth example of the plurality of kinds of signal amplification probes to be used in probe polymer formation. FIG. 11 is a schematic explanatory diagram illustrating a probe polymer to be formed using the plurality of kinds of signal amplification probes illustrated in FIGS. 10.

Examples of the plurality of kinds of signal amplification probes to be used in probe polymer formation include, as illustrated in FIGS. 10, one or more pairs of dimer formation probes, or one or more dimer probes to be formed from the pairs of dimer formation probes, and one or more kinds of cross-linking probes.

In FIGS. 10, there is illustrated an example of a case of using a pair of dimer formation probes and a pair of cross-linking probes.

In FIG. 10(a), there are illustrated a pair of dimer formation probes (a first dimer formation probe 31a and a second dimer formation probe 31b), and a dimer probe 30a formed from the pair of dimer formation probes 31a, 31b.

As illustrated in FIG. 10(a), the pair of dimer formation probes is formed of two kinds of single-stranded nucleic acid probes (the first dimer formation probe 31a and the second dimer formation probe 31b), and each of the dimer formation probes 31a, 31b includes at least three regions, i.e., a mid-region, a 5' side region located closer to the 5' side with respect to the mid-region, and a 3' side region located closer to the 3' side with respect to the mid-region. In FIGS. 10, the 5' side region, mid-region, and 3' side region of the first dimer formation probe 31a are represented by a region A, a region B, and a region C, respectively, and the 5' side region, mid-region, and 3' side region of the second dimer formation probe 31b are represented by a region D, a region B', and a region F, respectively. The mid-regions (regions B and B') of the first dimer formation probe 31a and the second dimer formation probe 31b are complementary to each other, and all of the four regions, i.e., the 5' side regions (regions A and D) and 3' side regions (regions C and F) of the first dimer formation probe 31a and the second dimer formation probe 31b are non-complementary to each other. Both the probes are allowed to hybridize with each other to form a dimer probe 30a.

In the present invention, a dimer probe formed by allowing a pair of dimer formation probes to hybridize with each other in advance may be used, or the dimer formation probes may be used as they are, but the dimer probe is preferably used.

In the case of using a plurality of pairs of dimer formation probes, each of the pairs of dimer formation probes is configured in the same manner as in the case of using one pair of dimer formation probes described above. The plurality of pairs of dimer formation probes are used to form the same number of dimer probes.

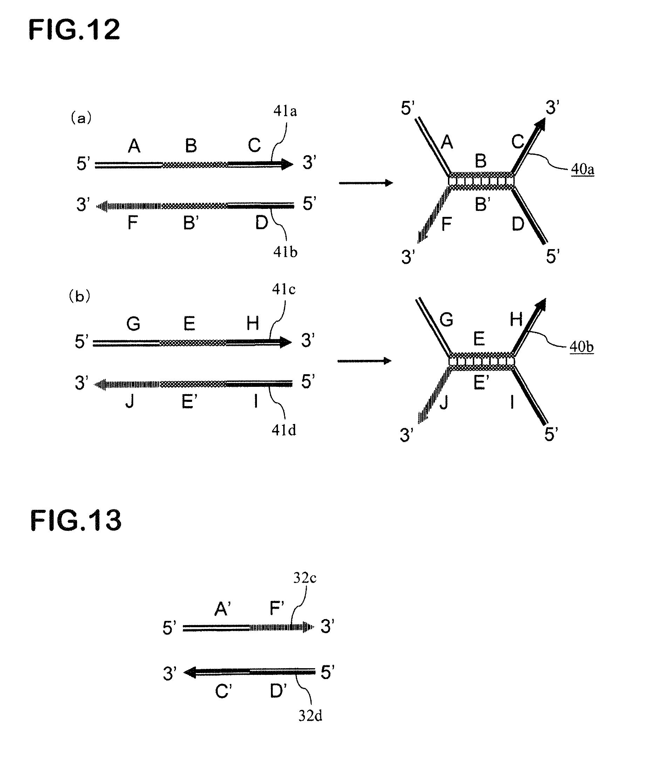

FIGS. 12 are schematic explanatory diagrams illustrating an example of two pairs of dimer formation probes, and two dimer probes to be formed from the pairs of dimer formation probes, in a fifth example of the plurality of kinds of signal amplification probes to be used in probe polymer formation. FIG. 12(a) illustrates a first pair of dimer formation probes (a first dimer formation probe 41a and a second dimer formation probe 41b), and a dimer probe 40a to be formed from the pair of dimer formation probes 41a, 41b, and FIG. 12(b) illustrates a second pair of dimer formation probes (a first dimer formation probe 41c and a second dimer formation probe 41d), and a dimer probe 40b to be formed from the pair of dimer formation probes 41c, 41d.

In FIGS. 12, the 5' side region, mid-region, and 3' side region of the first dimer formation probe 41a in the first pair are represented by a region A, a region B, and a region C, respectively, the 5' side region, mid-region, and 3' side region of the second dimer formation probe 41b in the first pair are represented by a region D, a region B', and a region F, respectively, the 5' side region, mid-region, and 3' side region of the first dimer formation probe 41c in the second pair are represented by a region G, a region E, and a region H, respectively, and the 5' side region, mid-region, and 3' side region of the second dimer formation probe 41d in the second pair are represented by a region I, a region E', and a region J, respectively. The mid-regions (regions B and B', or regions E and E') of the first and second dimer formation probes in each of the pairs are complementary to each other, all of the four regions (regions A, C, D, and F, and regions G, H, I, and J), i.e., the 5' side regions and 3' side regions of the first and second dimer formation probes in the respective pairs are non-complementary to each other, and the dimer formation probes 41a, 41b, 41c, 41d in each of the pairs are allowed to hybridize with each other to form the two dimer probes 40a, 40b. In the present invention, it is preferred that all of the 3' side regions and 5' side regions of the dimer formation probes to be used in PALSAR III be non-complementary to each other.

In the present invention, the cross-linking probes are one or more kinds of single-stranded nucleic acid probes that can cross-link the dimer probes to be formed from the dimer formation probes, and include at least two regions. Of the two regions, the region located on the 5' side is referred to as 5' side region, and the region located on the 3' side is referred to as 3' side region. In the present invention, each of the 5' side regions of the dimer formation probes is complementary to any one of the 5' side regions of the cross-linking probes, and each of the 3' side regions of the dimer formation probes is complementary to any one of the 3' side regions of the cross-linking probes. With such configuration, the cross-linking probes can bind to the dimer formation probes so as to cross-link one or more kinds of plurality of dimer probes to be formed from the dimer formation probes, to thereby form an assembly of probes (probe polymer).

In the case of using a pair of dimer formation probes, it is preferred to use a pair of cross-linking probes. The pair of dimer formation probes (a first dimer formation probe and a second dimer formation probe) and the pair of cross-linking probes (a first cross-linking probe and a second cross-linking probe) are configured so that: each of the 5' side regions of the pair of dimer formation probes is complementary to any one of the 5' side regions of the pair of cross-linking probes; each of the 5' side regions of the pair of cross-linking probes is complementary to any one of the 5' side regions of the pair of dimer formation probes; each of the 3' side regions of the pair of dimer formation probes is complementary to any one of the 3' side regions of the pair of cross-linking probes; and each of the 3' side regions of the pair of cross-linking probes is complementary to any one of the 3' side regions of the pair of dimer formation probes.

FIG. 10(b) is a schematic explanatory diagram illustrating an example of a pair of cross-linking probes (a first cross-linking probe 32a and a second cross-linking probe 32b) to be used together with the pair of dimer formation probes 31a, 31b illustrated in FIG. 10(a).

Suitable examples of the cross-linking probes to be used together with the pair of dimer formation probes 31a, 31b illustrated in FIG. 10(a) include, as illustrated in FIG. 10(b), such a pair of cross-linking probes that: the 5' side region (region A') of the first cross-linking probe 32a is complementary to the 5' side region (region A) of the first dimer formation probe 31a; the 3' side region (region C') of the first cross-linking probe 32a is complementary to the 3' side region (region C) of the first dimer formation probe 31a; the 5' side region (region D') of the second cross-linking probe 32b is complementary to the 5' side region (region D) of the second dimer formation probe 31b; and the 3' side region (region F') of the second cross-linking probe 32b is complementary to the 3' side region (region F) of the second dimer formation probe 31b. The dimer formation probes 31a, 31b illustrated in FIG. 10(a) and the cross-linking probes 32a, 32b illustrated in FIG. 10(b) can be allowed to hybridize with each other to form a probe polymer 34 (FIG. 11).

In FIGS. 10, there is illustrated an example in which: the 5' side region and 3' side region of the first dimer formation probe 31a are complementary to the 5' side region and 3' side region of the first cross-linking probe 32a, respectively; and the 5' side region and 3' side region of the second dimer formation probe 31b are complementary to the 5' side region and 3' side region of the second cross-linking probe 32b, respectively. In the present invention, such a configuration that the 5' side region of one dimer formation probe is complementary to the 5' side region of one cross-linking probe and the 3' side region of one dimer formation probe is complementary to the 3' side region of one cross-linking probe only needs to be adopted.

FIG. 13 is a schematic explanatory diagram illustrating another example of the pair of cross-linking probes (a first cross-linking probe 32c and a second cross-linking probe 32d) to be used together with the pair of dimer formation probes 31a, 31b illustrated in FIGS. 10.

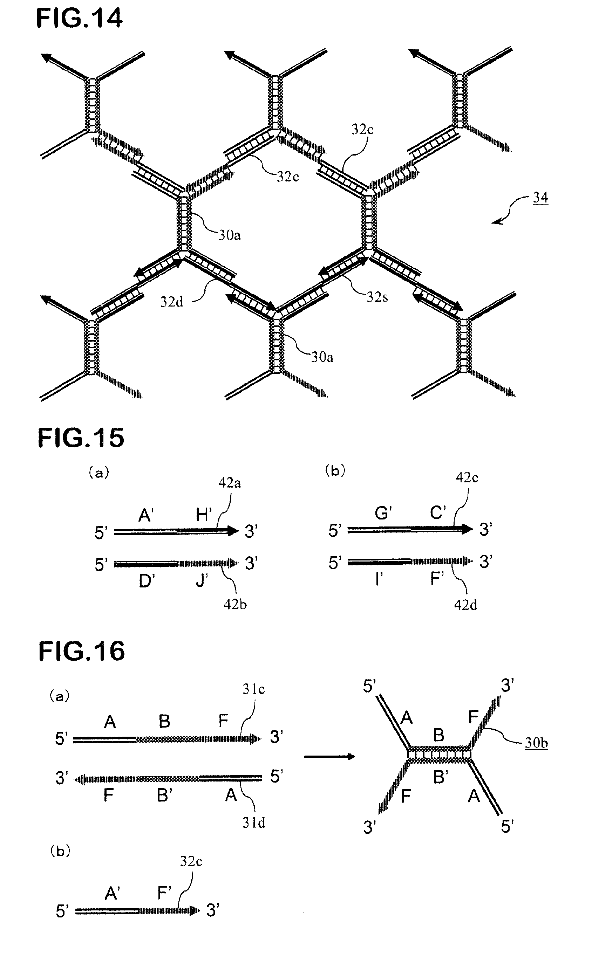

As illustrated in FIG. 13, as another example of the pair of cross-linking probes, there is given a pair of cross-linking probes in which: the 5' side region (region A') of the first cross-linking probe 32c is complementary to the 5' side region (region A) of the first dimer formation probe 31a; the 3' side region (region F') of the first cross-linking probe 32c is complementary to the 3' side region (region F) of the second dimer formation probe 31b; the 5' side region (region D') of the second cross-linking probe 32d is complementary to the 5' side region (region D) of the second dimer formation probe 31b; and the 3' side region (region C') of the second cross-linking probe 32d is complementary to the 3' side region (region C) of the first dimer formation probe 31b. The dimer formation probes 31a, 31b illustrated in FIG. 10(a) and the cross-linking probes 32c, 32d illustrated in FIG. 13 can be allowed to hybridize with each other to form a probe polymer 34 (FIG. 14).

In the case of using a plurality of pairs of dimer formation probes, the same number of pairs of cross-linking probes are preferably used. Specifically, n (n represents an integer of 2 or more) pairs of dimer formation probes (i.e., 2n dimer formation probes) and n pairs of cross-linking probes (i.e., 2n cross-linking probes) are used, and there is suitably adopted such a configuration that: each of the 5' side regions of the dimer formation probes is complementary to any one of the 5' side regions of the cross-linking probes; each of the 5' side regions of the cross-linking probes is complementary to any one of the other 5' side regions of the dimer formation probes; each of the 3' side regions of the dimer formation probes is complementary to any one of the 3' side regions of the cross-linking probes; and each of the 3' side regions of the cross-linking probes is complementary to any one of the other 3' side regions of the dimer formation probes.

FIGS. 15 are schematic explanatory diagrams illustrating an example of two pairs of cross-linking probes 42a to 42d to be used together with the two pairs of dimer formation probes 41a to 41d illustrated in FIGS. 12. FIG. 15(a) illustrates a first pair of cross-linking probes (the first cross-linking probe 42a and the second cross-linking probe 42b), and FIG. 15(b) illustrates a second pair of cross-linking probes (the first cross-linking probe 42c and the second cross-linking probe 42d).

As illustrated in FIGS. 15, two pairs of cross-linking probes (i.e., four kinds of cross-linking probes) are used as cross-linking probes to be used together with the two pairs of dimer formation probes 41a to 41d illustrated in FIGS. 12, and there is suitably adopted such a configuration that: each of the 5' side regions of the dimer formation probes is complementary to any one of the 5' side regions of the cross-linking probes; each of the 5' side regions of the cross-linking probes is complementary to any one of the 5' side regions of the dimer formation probes; each of the 3' side regions of the dimer formation probes is complementary to any one of the 3' side regions of the cross-linking probes; and each of the 3' side regions of the cross-linking probes is complementary to any one of the 3' side regions of the dimer formation probes.

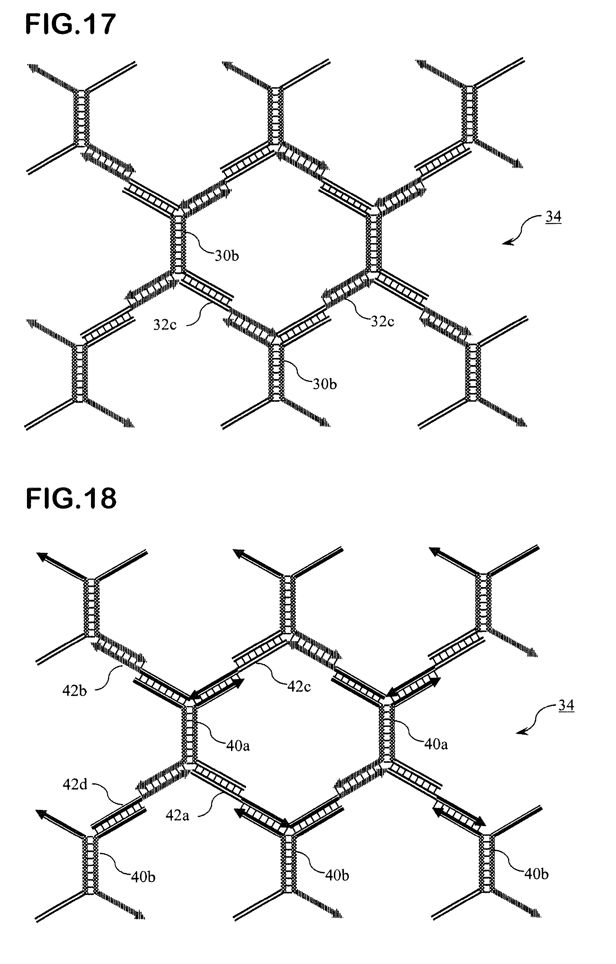

Specifically, as illustrated in FIGS. 15, there are suitably used two pairs of cross-linking probes in which: the 5' side region (region A') of the first cross-linking probe 42a in the first pair is complementary to the 5' side region (region A) of the first dimer formation probe 41a in the first pair; the 5' side region (region D') of the second cross-linking probe 42b in the first pair is complementary to the 5' side region (region D) of the second dimer formation probe 41b in the first pair; the 5' side region (region G') of the first cross-linking probe 42c in the second pair is complementary to the 5' side region (region G) of the first dimer formation probe 41c in the second pair; the 5' side region (region I') of the second cross-linking probe 42d in the second pair is complementary to the 5' side region (region I) of the second dimer formation probe 41d in the second pair; the 3' side region of the first cross-linking probe 42a in the first pair is complementary to any one of the 3' side regions of the four kinds of dimer formation probes 41a to 41d (in FIGS. 15, (region H') is illustrated a case of being complementary to the 3' side region (region H) of the first dimer formation probe 41c in the second pair); the 3' side region of the second cross-linking probe 42b in the first pair is complementary to any one of the 3' side regions of the four kinds of dimer formation probes 41a to 41d, except for the one selected for the first cross-linking probe 42a in the first pair (in FIGS. 15, t(region J') is illustrated a case of being complementary to the 3' side region (region J) of the second dimer formation probe 41d in the second pair); the 3' side region of the first cross-linking probe 42c in the second pair is complementary to any one of the 3' side regions of the four kinds of dimer formation probes 41a to 41d, except for the ones selected for the first and second cross-linking probes 42a and 42b in the first pair (in FIGS. 15, (region C') is illustrated a case of being complementary to the 3' side region (region C) of the first dimer formation probe 41a in the first pair); and the 3' side region of the second cross-linking probe 42d in the second pair is complementary to one of the 3' side regions of the four kinds of dimer formation probes 41a to 41d, except for the ones selected for the first and second cross-linking probes 42a, 42b in the first pair and the first cross-linking probe 42c in the second pair (in FIGS. 15, (region F') is illustrated a case of being complementary to the 3' side region (region F) of the second dimer formation probe 41b in the first pair). In this regard, however, the first pair of cross-linking probes is never the same as any of the cross-linking probes 32a to 32d illustrated in FIGS. 10 and FIG. 13. The dimer formation probes 41a to 41d illustrated in FIGS. 12 can be allowed to hybridize with the cross-linking probes 42a to 42d illustrated in FIGS. 15 to form a probe polymer 34 (FIG. 18).

It should be noted that in FIGS. 15, there is illustrated a case where: the 3' side region of the first cross-linking probe 42a in the first pair is complementary to the 3' side region of the first dimer formation probe 41c in the second pair; the 3' side region of the second cross-linking probe 42b in the first pair is complementary to the 3' side region of the second dimer formation probe 41b in the second pair; the 3' side region of the first cross-linking probe 42c in the second pair is complementary to the 3' side region of the first dimer formation probe 41a in the first pair; and the 3' side region of the second cross-linking probe 42d in the second pair is complementary to the 3' side region of the second dimer formation probe 41b in the first pair, but no limitation is imposed on a combination of the 3 side regions of the dimer formation probes to be complementary to each of the 3' side regions of the cross-linking probes.

FIGS. 16 are schematic explanatory diagrams illustrating a sixth example of the plurality of kinds of signal amplification probes to be used in probe polymer formation, and are schematic explanatory diagrams illustrating an example of a pair of dimer formation probes and one kind of pair of cross-linking probes. In FIG. 16(a), reference symbol 30b denotes a dimer probe, and there is illustrated an example of dimer formation using two kinds of dimer formation probes 31c, 31d in which the 3' side regions and the 5' side regions each have identical base sequences. That is, there is adopted such a configuration that in the dimer probe 30a of FIGS. 10, the region A and the region D have identical base sequences (represented by the region A in FIGS. 16), and the region C and the region F have identical base sequences (represented by the region F in FIGS. 16). With this configuration, as illustrated in FIG. 16(b), the pair of cross-linking probes to be used together with the dimer probe 30b are identical to each other, and one kind of cross-linking probe 32c is used. The dimer formation probes 31c, 31d illustrated in FIGS. 16 and the cross-linking probe 32c can be allowed to hybridize with each other to form a probe polymer 34 (FIG. 17).

In the plurality of kinds of signal amplification probes to be used in probe polymer formation of the present invention, the length of each complementary region of each probe is 5 bases or more, preferably 8 bases or more, more preferably from 10 bases to 100 bases, still more preferably from 12 to 30 bases in terms of the number of bases. In addition, the lengths of the complementary regions in the respective probes are desirably the same.

The base sequence of each region of the plurality of kinds of signal amplification probes to be used in probe polymer formation of the present invention is not particularly limited as long as predetermined regions are configured to have complementary base sequences so as to form a probe polymer, but bases at both ends of each region are each preferably guanine or cytosine. When the bases at both ends of each region are each guanine or cytosine, a reaction time can be shortened, a stable probe polymer can be formed at additionally low reaction temperature, and workability and detection sensitivity can be improved.

The signal amplification probes are generally constructed of DNA or RNA, but may be constructed of a nucleic acid analog. Examples of the nucleic acid analog include a peptide nucleic acid (PNA, see, for example, WO 92/20702 A1) and a locked nucleic acid (LNA, see, for example, Koshkin AA et al. Tetrahedron1998.54, 3607-3630., Koshkin AA et al. J. Am. Chem. Soc. 1998.120, 13252-13253., Wahlestedt C et al. PNAS.2000.97, 5633-5638.). In addition, the plurality of kinds of signal amplification probes are generally constructed of nucleic acids of the same kind, but for example, a DNA probe and an RNA probe may form a pair. That is, the kind of a nucleic acid of a probe may be selected from DNA, RNA, and a nucleic acid analog (e.g., PNA or LNA). In addition, for nucleic acid composition in one probe, the probe does not need to be constructed of one kind of nucleic acid, for example, DNA alone, and for example, an oligonucleotide probe (chimeric probe) constructed of DNA and RNA may also be used as necessary, which is also included in the present invention.

Such probe may be synthesized by a known method. For example, the DNA probe may be synthesized by a phosphoramidite method using a DNA synthesizer Model 394 manufactured by Applied Biosystems Inc. In addition, a phosphoric acid triester method, an H-phosphonate method, a thiophosphonate method, or the like is available as an alternative method, and the probe may be synthesized by any method.

As the plurality of kinds of signal amplification probes, ones labeled with a labeling substance are preferably used. Suitable examples of the labeling substance include a radioisotope, biotin, digoxigenin, a fluorophore, a luminophore, and a pigment. Preferred examples of the labeling substance may include Cy3, Alexa 532, and biotin.

The detection method for a nucleotide mutation of the present invention is a method including using the detection kit of the present invention, the method including the following steps (a) to (c):
(a) amplifying a nucleic acid to be tested through an amplification reaction to obtain a DNA amplification product;
(b) subjecting the DNA amplification product, a capture probe (first nucleic acid probe), an assist probe (second nucleic acid probe), and a plurality of kinds of signal amplification probes to a reaction to form a complex for detection including the DNA amplification product, the capture probe (first nucleic acid probe), the assist probe (second nucleic acid probe), and a probe polymer formed from the plurality of kinds of signal amplification probes; and
(c) detecting the probe polymer bound in the complex for detection to detect the presence or absence of a mutation in a nucleotide mutation site of the DNA amplification product.

In Step (a), the nucleic acid to be tested refers to DNA and RNA. The amplification reaction is not particularly limited as long as the reaction amplifies the nucleic acid to be tested to obtain a DNA amplification product, but is suitably a polymerase chain reaction (PCR).

The DNA amplification product is preferably converted into a single-stranded one through a denaturation operation after Step (a) and before Step (b). A method of denaturing a multiple-stranded nucleic acid into a single-stranded one is suitably a method based on heating. The denaturation of double-stranded DNA, which also depends on conditions such as the components and pH of a nucleic acid solution, is generally caused by heating under physiological conditions at 90°C or more for from several seconds to several minutes. After that, when the temperature is reduced to a predetermined one in the presence of the capture probe and the assist probe, the capture probe and the assist probe can be allowed to hybridize with the DNA amplification product denatured into a single-stranded one. In addition, alkali treatment is also effective for the denaturation of the multiple-stranded nucleic acid, but requires neutralization treatment for allowing the capture probe and the assist probe to hybridize with the denatured DNA amplification product. In addition, a method involving using a chemical substance having a denaturation action, such as formamide, is available, but such chemical substance is toxic and hence needs to be carefully handled. From those viewpoints, the denaturation by heating is simple and general.

In Step (b), as illustrated in FIGS. 1, there may occur a case where bases are completely matched with each other [FIG. 1(a)] and a case where a mismatched base pair is contained [FIG. 1(b)] in a hybrid formed by the hybridization between the capture probe 12 and the DNA amplification product 10 based on a difference in kind of a base in the nucleotide mutation site Tx of the DNA amplification product 10.

It should be noted that in FIGS. 1 to 3, there is illustrated an example of, after Step (b1) of subjecting a DNA amplification product, a capture probe, and an assist probe to a reaction, subjecting a plurality of kinds of signal amplification probes to a reaction to form a probe polymer [Step (b2)] to form a complex for detection including the DNA amplification product, the capture probe, the assist probe, and the probe polymer, but in the present invention, reactions among the DNA amplification product, the capture probe, the assist probe, and the plurality of kinds of signal amplification probes may be simultaneously performed, or may be separately performed in a plurality of stages, without any particular limitation. In addition, when the reactions are separately performed in a plurality of stages, their order is also not particularly limited.

In the present invention, a probe polymer formation reaction is performed immediately after the hybridization between the capture probe and the DNA amplification product without performing B/F separation by washing, or simultaneously with the hybridization between the DNA amplification product, and the capture probe and the assist probe. Thus, the sensitivity and specificity of detection of a complete match and mismatch present between the capture probe and the DNA amplification product can be drastically improved.

That is, when Step (b) is performed, a huge complex for detection formed of the solid phase, the capture probe, the DNA amplification product, the assist probe, and a probe polymer formed from the signal amplification probes is formed. When the probe polymer increases in size along with the progress of the self-assembly reaction, the thermodynamic energy of the complex increases, resulting in an unstable state. Soon later, when the energy of the complex excessively increases, transition to a more stable state occurs, and hence a portion whose binding is weakest disintegrates. A binding having a mismatch between the capture probe and the DNA amplification product has the weakest binding force in such complex. As described above, when a mismatch is present between the capture probe and the DNA amplification product, the capture probe and the DNA amplification product dissociate from each other, and hence significant signal amplification by the probe polymer is not detected in the solid phase [FIG. 2(c)]. On the other hand, a completely complementary binding between the capture probe and the DNA amplification product keeps a sufficient binding force at a predetermined temperature even when energy increases through the formation of a self-assembly, and hence the complex formed of the solid phase, the capture probe, the DNA amplification product, the assist probe, and the probe polymer formed from the signal amplification probes is stably maintained. As a result, significant signal amplification by the probe polymer can be detected [FIG. 2(a)].

Thus, the presence or absence of a mutation in the nucleotide mutation site Tx of the DNA amplification product can be detected by detecting the probe polymer bound in the complex for detection [Step (c)]. A detection method for the probe polymer is not particularly limited, and the probe polymer is suitably detected by fluorescence, light emission, color development, or the like.

### Examples

The present invention is more specifically described below by way of Examples. However, it should be appreciated that Examples shown below are given for illustrative purposes and should not be interpreted as limiting the present invention.

### (Example 1)

SNP detection was performed by the detection method for a mutation gene of the present invention using a polymorphism of a nucleotide at position 857 of N-acetyltransferase 2 (NAT2) gene as a target.

Two kinds of alleles, i.e., guanine (G) and adenine (A) alleles are present at position 857 of the NAT2 gene. Thus, part of regions of the NAT2 gene including the position 857 was amplified by a PCR, and an experiment for assessing the type of the SNP was performed using its PCR product as a target.

A region of about 700 bases including an SNP site at position 857 of NAT2 was amplified from a human genome specimen by a PCR. Sequences of primer DNAs used in the PCR are shown below.
5'-CGGTTTTCAGACCACAATGTTAGGA-3' (SEQ ID NO: 1)
5'-TGAGTTGGGTGATACATACACAAGG-3' (SEQ ID NO: 2)

The composition of the PCR reaction solution is as follows: 1X Phusion High-Fidelity DNA Polymerase Buffer (Finnzymes); Phusion High-Fidelity DNA Polymerase: 0.02 U/µL; each primer DNA: 500 nM; each dNTP: 200 nM; and human genome DNA: 1 to 10 ng/µL, and the volume is 10 /µL. epGradient S manufactured by Eppendorf Co., Ltd. was used as a thermal cycler. As a PCR reaction, a cycle of a denaturation reaction at 98°C for 30 seconds, followed by 99°C for 5 seconds, 62°C for 15 seconds, and 72°C for 15 seconds was performed 40 times, and finally an extension reaction at 72°C for 1 minute was performed. The confirmation of whether or not the PCR reaction was successful was performed as follows: 2 µL of the PCR product were subjected to electrophoresis with 2% agarose (Takara Bio Inc.)/1X TAE buffer, then stained with ethidium bromide, and visualized by irradiation with UV light at 365 nm.

In order to assess the SNP at position 857 of the NAT2 gene, two kinds of capture probes formed of a first target sequence region complementary to a first region of a PCR amplification product including an SNP site at the center and having the following base sequences were used as a first nucleic acid probe. The 5' end of each of the probes has been modified with an amino group in order to allow the probes to covalently bind to a solid phase.
Base sequence of capture probe for allele G detection (5'-T₁(G)'-3')
5'-NH₂-ACCTGGTGATG G ATCCCTTAC-3'(SEQ ID NO: 3)
Base sequence of capture probe for allele A detection (5'-T₁(A)'-3')
5'-NH₂-ACCTGGTGATG A ATCCCTTAC-3' (SEQ ID NO: 4)

The immobilization of the capture probe onto the solid phase was performed by the following method. xMAP fine particles manufactured by Luminex were used as the solid phase. The xMAP fine particles have been modified with a carboxyl group, and thus the immobilization can be performed through a coupling reaction with an aminated oligonucleotide. 200 µL of the xMAP fine particles at 12,500,000 particles/mL (equivalent to 2,500,000 particles) were loaded into a 1.5-mL microcentrifugation tube, and centrifuged (10,000 rpm, 5 minutes) to remove the supernatant liquid. Next, 25 µL of 0.1 M MES buffer at pH 4.5 were added to the fine particles. To the mixture were added 100 pmol of the capture probe and 10 µL of 1-ethyl-3-[3-dimethylaminopropyl]carbodiimide hydrochloride (EDC) (Theromo Scientific) dissolved at 10 mg/mL with a 2-morpholinoethanesulfonic acid (MES) solution. The resultant was sonicated at 45 kHz for several seconds using an ultrasonic cleaning device US-100 III manufactured by AS ONE Corporation to completely disperse the fine particles. The dispersion was left to stand in the dark at room temperature (25°C) for 30 minutes, then 10 µL of 10 mg/mL EDC were added, and the mixture was sonicated for several seconds. Further, the resultant was left to stand in the dark at room temperature for 30 minutes. After that, 0.5 mL of 0.02% Tween 20 was added, and the mixture was vortexed for several seconds and then centrifuged (10,000 rpm, 3 minutes) to remove the supernatant liquid. Next, 0.5 mL of 0.1% SDS was added, and the mixture was vortexed for several seconds and then centrifuged (10,000 rpm, 3 minutes) to remove the supernatant liquid. To the liquid was added an appropriate amount of TE buffer, and the mixture was sonicated for several seconds for dispersion. The concentration of the fine particles was measured with a Burker-Turk hemacytometer, and adjusted to a concentration of 10,000 particles/mL with TE buffer.

It should be noted that the capture probe for allele G detection and the capture probe for allele A detection were immobilized onto the xMAP fine particles having ID numbers different from each other. A difference in color tone of a fluorescent dye applied onto the fine particles having ID numbers different from each other is distinguished with a flow cytometer manufactured by Luminex, and a target substance-specific signal can be measured based on the distinction.

A first signal amplification probe [HCP-1] labeled at the 5' end with Cy3 and a second signal amplification probe [HCP-2] labeled at the 5' end with Cy3 were used as a plurality of kinds of signal amplification probes. Base sequence of HCP-1 (5'-X-Y-Z-3')
5'-CATCTCTGCTGGTC-CCACATTCAGACCC-GTTCGCCATAGACG-3' (SEQ ID NO: 5)
Base sequence of HCP-2 (5'-X'-Y'-Z'-3')
5'-GACCAGCAGAGATG-GGGTCTGAATGTGG-CGTCTATGGCGAAC-3' (SEQ ID NO: 6)

An assist probe-1 having the following base sequence was used as a second nucleic acid probe. The assist probe-1 is a nucleic acid probe having a region complementary to the PCR amplification product and a base sequence identical to part of the base sequence of HCP-1 (sequence ZYZ), and is configured so as to hybridize with the PCR amplification product in a state in which the capture probe and the assist probe-1 are adjacent to each other. Base sequence of assist probe-1 (5'-T₂(0)'-Z-Y-Z-3')

5'-TATTTAGAATAAGGAA-GTTCGCCATAGACG CCACATTCAGACCC
GTTCGCCATAGACG-3' (SEQ ID NO: 7)

A reaction solution having a volume of 25 µL and containing 0.2 µL of xMAP fine particles onto which the capture probe for allele G detection had been immobilized at 10,000 particles/µL, 0.2 µL of xMAP fine particles onto which the capture probe for allele A detection had been immobilized at 10,000 particles/µL, 0.25 µL of 1 µM assist probe-1, 0.625 µL of 20 µM HCP-1, 0.75 µL of 20 µM HCP-1, 1 µL of the PCR amplification product, 7.5 µL of 5 M tetramethylammonium chloride (TMAC) buffer, 2.5 µL of 10×Supplement [1 M N-lauroylsarcosine, 500 mM Tris-HCl (pH 8.0), 40 mM EDTA], and 11.975 µL of sterile distilled water was prepared in a PCR reaction tube (volume: 0.2 mL).

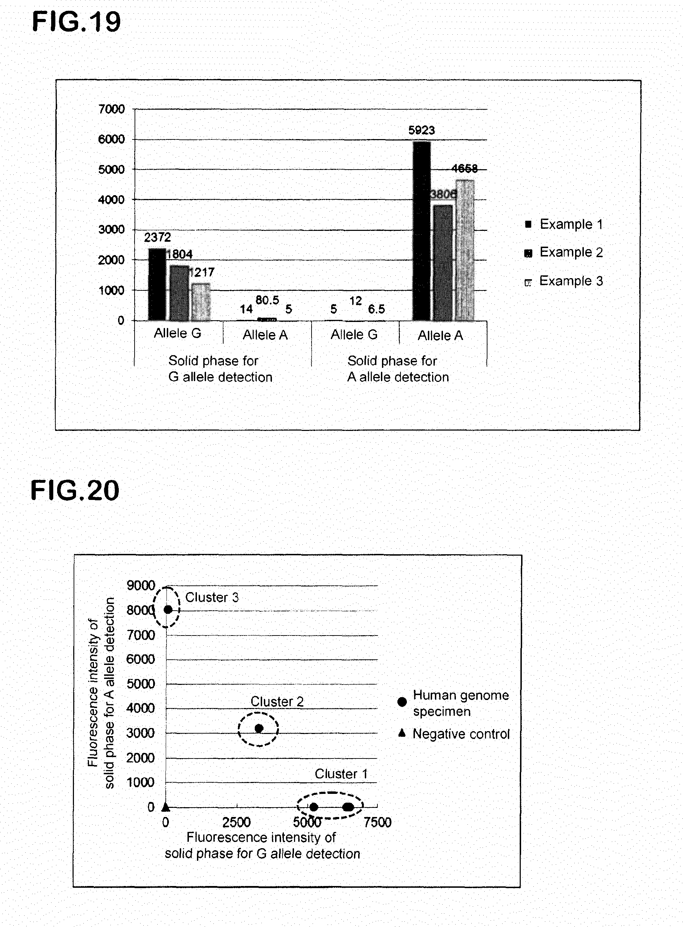

The PCR reaction tube was mounted onto a thermal cycler (epGradient S manufactured by Eppendorf), and a program of 95°C for 5 minutes (denaturation reaction of a PCR product) and 58°C for 60 minutes [Step (b)] was carried out. After the completion of the reaction, each reaction solution was transferred to wells of a MultiScreen HTS plate (Millipore), and the liquid was removed by suction filtration. 200 µL of TP/PBS were added to each well, and the liquid was removed by suction again to wash the solid phase. Further, 100 µL of TP/PBS were added, and the mixture was shaken with a plate mixer for about 30 seconds to sufficiently disperse the solid phase. After that, the MultiScreen HTS plate was mounted onto Luminex 100, and the fluorescence intensity of the solid phase in each well was measured. FIG. 19 shows the results.

### (Example 2)

An experiment was performed in the same manner as in Example 1 except that an assist probe-2 having the following base sequence was used in place of the assist probe-1 as the second nucleic acid probe. The assist probe-2 is a nucleic acid probe having a region complementary to the PCR amplification product and a base sequence identical to part of the base sequence of HCP-1 (sequence ZYZ), and is configured so that the capture probe and the assist probe-2 hybridize with the PCR amplification product in a state in which the probes are located away from each other by 1 base. FIG. 19 shows the results.
Base sequence of assist probe-2 (5'-T₂(1)'-Z-Y-Z-3')

5'-ATTTAGAATAAGGAAC-GTTCGCCATAGACG CCACATTCAGACCC
GTTCGCCATAGACG-3' (SEQ ID NO: 8)

### (Example 3)

An experiment was performed in the same manner as in Example 1 except that an assist probe-3 having the following base sequence was used in place of the assist probe-1 as the second nucleic acid probe. The assist probe-3 is a nucleic acid probe having a region complementary to the PCR amplification product and a base sequence identical to part of the base sequence of HCP-1 (sequence ZYZ), and is configured so that the capture probe and the assist probe-3 hybridize with the PCR amplification product in a state in which the probes are located away from each other by 2 bases. FIG. 19 shows the results.
Base sequence of assist probe-3 (5'-T₂(2)'-Z-Y-Z-3')

5'-TTTAGAATAAGGAACA-GTTCGCCATAGACG CCACATTCAGACCC

GTTCGCCATAGACG-3' (SEQ ID NO: 9)

As shown in FIG. 19, an SNP was able to be detected with high sensitivity by the method of the present invention. In addition, a highly allele-specific signal was obtained in any of Example 1, in which the first nucleic acid probe and the second nucleic acid probe hybridize with the DNA amplification product in a state in which the probes are adjacent to each other, and Examples 2 and 3, in which the first nucleic acid probe and the second nucleic acid probe hybridize with the DNA amplification product in a state in which the probes are not adjacent to each other.

### (Example 4)

An experiment was performed in the same manner as in Example 1 using five kinds of commercially available human tissue-derived genome DNA specimens. FIG. 20 shows the results. FIG. 20 shows scatter plot data in which the abscissa axis indicates the fluorescence intensity of the solid phase for G allele detection and the ordinate axis indicates the fluorescence intensity of the solid phase for A allele detection. Based on the results, those five kinds of specimens are classified into three clusters: a cluster in which only the fluorescence intensity of the solid phase for G allele detection is high (Cluster 1); a cluster in which the solid phases for G allele and A allele detection are both high (Cluster 2); and a cluster in which only the fluorescence intensity of the solid phase for allele detection is high (Cluster 3). When a base at position 857 of the NAT2 gene of those five specimens was determined by a direct sequencing method, the specimen of Cluster 1 was G-homo, the specimen of cluster 2 was G/A-hetero, and the specimen of Cluster 3 was A-homo. The results of SNP typing by the sequencing method and the method of the present invention were exactly the same. This showed that the assessment of the SNP is properly performed by the present invention.

### Description of Symbols

10: DNA amplification product, 12: first nucleic acid probe, capture probe, 13: support, 14: second nucleic acid probe, assist probe, 16: signal amplification probe, 16a: first signal amplification probe, 16b: second signal amplification probe, 18, 22, 34: probe polymer, 20a: first dimer probe, 20b, 20c, 20d: second dimer probe, 20e: third dimer probe, 21a to 21h, 21j, 21k: dimer formation probe, 30a, 30b: dimer probe, 31a to 31d: dimer formation probe, 32a to 32d: cross-linking probe, 40a, 40b: dimer probe, 41a to 41d: dimer formation probe, 42a to 42d: cross-linking probe, Tx: nucleotide mutation site.

## Claims

1. A detection method for a nucleotide mutation, comprising the steps of:
(a) amplifying a nucleic acid to be tested through an amplification reaction to obtain a DNA amplification product;
(b) subjecting
the DNA amplification product,
a first nucleic acid probe having a first target sequence region complementary to a first region of the DNA amplification product,
a second nucleic acid probe having a second target sequence region complementary to a second region of the DNA amplification product, and
a plurality of kinds of signal amplification probes having complementary base sequence regions capable of hybridizing with each other and being capable of forming a probe polymer through a self-assembly reaction
to a reaction to form a complex for detection comprising the DNA amplification product, the first nucleic acid probe, the second nucleic acid probe, and the probe polymer formed from the plurality of kinds of signal amplification probes; and
(c) detecting the probe polymer bound in the complex for detection to detect a presence or absence of a mutation in a nucleotide mutation site of the DNA amplification product,
the first region of the DNA amplification product comprising the nucleotide mutation site, the nucleotide mutation site being located at a portion other than both end portions of the first region of the DNA amplification product,
the second nucleic acid probe having a base sequence identical to part or all of a base sequence of one of the plurality of kinds of signal amplification probes.

2. A detection method for a nucleotide mutation according to claim 1, wherein:
the first nucleic acid probe has the first target sequence region at a 5' end or a 3' end; and
the second nucleic acid probe has the second target sequence region at a 3' end or a 5' end.

3. A detection method for a nucleotide mutation according to claim 1 or 2, wherein the first nucleic acid probe is immobilized onto a support or has introduced therein a moiety capable of being immobilized onto a support.

4. A detection method for a nucleotide mutation according to any one of claims 1 to 3, wherein at least one of the plurality of kinds of signal amplification probes is labeled with a labeling substance.

5. A detection method for a nucleotide mutation according to any one of claims 1 to 4, wherein:
the plurality of kinds of signal amplification probes include a first signal amplification probe and a second signal amplification probe;
the first signal amplification probe comprises a nucleic acid probe comprising three or more nucleic acid regions that comprise at least a nucleic acid region X, a nucleic acid region Y, and a nucleic acid region Z in the stated order from a 5' end side; and
the second signal amplification probe comprises a nucleic acid probe comprising three or more nucleic acid regions that comprise at least a nucleic acid region X' complementary to the nucleic acid region X, a nucleic acid region Y' complementary to the nucleic acid region Y, and a nucleic acid region Z' complementary to the nucleic acid region Z in the stated order from a 5' end side.

6. A detection method for a nucleotide mutation according to any one of claims 1 to 4, wherein:
the plurality of kinds of signal amplification probes comprise a plurality of dimer probes or dimer formation probes for forming the dimer probes;
each of the plurality of dimer probes comprises a dimer formed from two kinds of dimer formation probes;
each of the dimer formation probes comprises three regions, i.e., a 5' side region, a mid-region, and a 3' side region;
in the two kinds of dimer formation probes for forming the dimer, the mid-regions are complementary to each other and the 3' side regions and the 5' side regions are non-complementary to each other; and
each of the 5' side regions of each of the plurality of dimer probes is complementary to any one of the 5' side regions of other dimer probes and each of the 3' side regions of each of the dimer probes is complementary to any one of the 3' side regions of the other dimer probes.

7. A detection method for a nucleotide mutation according to any one of claims 1 to 4, wherein:
the plurality of kinds of signal amplification probes include
one or more pairs of dimer formation probes or one or more dimer probes to be formed from the pairs of dimer formation probes, and
one or more kinds of cross-linking probes;
each of the dimer formation probes comprises three regions, i.e., a 5' side region, a mid-region, and a 3' side region, the mid-regions of each of the pairs of dimer formation probes are complementary to each other, and all of the 3' side regions and 5' side regions of each of the pairs of dimer formation probes are non-complementary to each other;
each of the cross-linking probes comprises two regions, i.e., a 5' side region and a 3' side region; and
each of the 5' side regions of the dimer formation probes is complementary to any one of the 5' side regions of the cross-linking probes, and each of the 3' side regions of the dimer formation probes is complementary to any one of the 3' side regions of the cross-linking probes.

8. A detection kit to be used for detection of a nucleotide mutation in a DNA amplification product obtained by amplification through a polymerase chain reaction,
the detection kit comprising:
a first nucleic acid probe;
a second nucleic acid probe; and
a plurality of kinds of signal amplification probes having complementary base sequence regions capable of hybridizing with each other and being capable of forming a probe polymer through a self-assembly reaction,
wherein:
the first nucleic acid probe has a first target sequence region complementary to a first region of the DNA amplification product;
the second nucleic acid probe has a second target sequence region complementary to a second region of the DNA amplification product;
the first region of the DNA amplification product comprises a nucleotide mutation site, the nucleotide mutation site being located at a portion other than both end portions of the first region of the DNA amplification product; and
the second nucleic acid probe has a base sequence identical to part or all of a base sequence of one of the plurality of kinds of signal amplification probes.

9. A detection kit according to claim 8, wherein the first nucleic acid probe is immobilized onto a support or has introduced therein a moiety capable of being immobilized onto a support.

10. A detection kit according to claim 8 or 9, wherein at least one of the plurality of kinds of signal amplification probes is labeled with a labeling substance.

11. A detection kit according to any one of claims 8 to 10, wherein:
the plurality of kinds of signal amplification probes include a first signal amplification probe and a second signal amplification probe;
the first signal amplification probe comprises a nucleic acid probe comprising three or more nucleic acid regions that comprise at least a nucleic acid region X, a nucleic acid region Y, and a nucleic acid region Z in the stated order from a 5' end side; and
the second signal amplification probe comprises a nucleic acid probe comprising three or more nucleic acid regions that comprise at least a nucleic acid region X' complementary to the nucleic acid region X, a nucleic acid region Y' complementary to the nucleic acid region Y, and a nucleic acid region Z' complementary to the nucleic acid region Z in the stated order from a 5' end side.

12. A detection kit according to any one of claims 8 to 10, wherein:
the plurality of kinds of signal amplification probes comprise a plurality of dimer probes or dimer formation probes for forming the dimer probes;
each of the plurality of dimer probes comprises a dimer formed from two kinds of dimer formation probes;
each of the dimer formation probes comprises three regions, i.e., a 5' side region, a mid-region, and a 3' side region;
in the two kinds of dimer formation probes for forming the dimer, the mid-regions are complementary to each other and the 3' side regions and the 5' side regions are non-complementary to each other; and
each of the 5' side regions of each of the plurality of dimer probes is complementary to any one of the 5' side regions of other dimer probes and each of the 3' side regions of each of the dimer probes is complementary to any one of the 3' side regions of the other dimer probes.

13. A detection detection kit according to any one of claims 8 to 10, wherein:
the plurality of kinds of signal amplification probes include
one or more pairs of dimer formation probes or one or more dimer probes to be formed from the pairs of dimer formation probes, and
one or more kinds of cross-linking probes;
each of the dimer formation probes comprises three regions, i.e., a 5' side region, a mid-region, and a 3' side region, the mid-regions of each of the pairs of dimer formation probes are complementary to each other, all of the 3' side regions and 5' side regions of each of the pairs of dimer formation probes are non-complementary to each other;
each of the cross-linking probes comprises two regions, i.e., a 5' side region and a 3' side region; and
each of the 5' side regions of the dimer formation probes is complementary to any one of the 5' side regions of the cross-linking probes, and each of the 3' side regions of the dimer formation probes is complementary to any one of the 3' side regions of the cross-linking probes.


SEQUENCE LISTING

<110>  Eisai R&D Management Co., Ltd.

<120>  Method for detecting nucleotide variants and detection kit

<130>  77888-P-PCT

<150>  JP 2011-269967
<151>  2011-12-09

<160>  9

<210>  1
<211>  25
<212>  DNA
<213>  Artificial

<220>
<223>  Description of Artificial Sequence; Synthetic

<400>  1
cggttttcag accacaatgt tagga                                          25


<210>  2
<211>  25
<212>  DNA
<213>  Artificial

<220>
<223>  Description of Artificial Sequence; Synthetic

<400>  2
tgagttgggt gatacataca caagg                                          25


<210>  3
<211>  21
<212>  DNA
<213>  Artificial

<220>
<223>  Description of Artificial Sequence; Synthetic

<220>
<221>  misc_feature
<222>  (1)..(1)
<223>  Amino group attached at the 5'end


<400>  3
acctggtgat ggatccctta c                                              21


<210>  4
<211>  21
<212>  DNA
<213>  Artificial

<220>
<223>  Description of Artificial Sequence; Synthetic

<220>
<221>  misc_feature
<222>  (1)..(1)
<223>  Amino group attached at the 5'end


<400>  4
acctggtgat gaatccctta c                                                21


<210>  5
<211>  42
<212>  DNA
<213>  Artificial

<220>
<223>  Description of Artificial Sequence; Synthetic

<400>  5
catctctgct ggtcccacat tcagacccgt tcgccataga cg                         42


<210>  6
<211>  42
<212>  DNA
<213>  Artificial

<220>
<223>  Description of Artificial Sequence; Synthetic

<400>  6
gaccagcaga gatggggtct gaatgtggcg tctatggcga ac                         42


<210>  7
<211>  58
<212>  DNA
<213>  Artificial

<220>
<223>  Description of Artificial Sequence; Synthetic

<400>  7
tatttagaat aaggaagttc gccatagacg ccacattcag acccgttcgc catagacg        58


<210>  8
<211>  58
<212>  DNA
<213>  Artificial

<220>
<223>  Description of Artificial Sequence; Synthetic

<400>  8
atttagaata aggaacgttc gccatagacg ccacattcag acccgttcgc catagacg        58


<210>  9
<211>  58
<212>  DNA
<213>  Artificial

<220>
<223> Description of Artificial Sequence; Synthetic

<400> 9
tttagaataa ggaacagttc gccatagacg ccacattcag acccgttcgc catagacg 58